# EUROPEAN PATENT APPLICATION

(11) **EP 2 950 102 A1**
(43) Date of publication of application: **02.12.2015**
(21) Application number: 14382203.9
(22) Date of filing: 30.05.2014
(51) Int. Cl.: G01N 33/68

(54) **Method for the diagnosis of alzheimer s disease and mild cognitive impairment**

(71) Applicant: Biocross, S.L., 47151 Boecillo (ES)
(72) Inventor: Zanotti, Carlo, 47151 Boecillo (ES); Rodríguez Mártin, Andrés, 47151 Boecillo (ES); Gil De Gómez Sesma, Luis, 47151 Boecillo (ES)
(74) Representative: ABG Patentes, S.L.

(57) **Abstract**

The invention relates to methods for determining the likelihood that a patient with mild cognitive impairment develops Alzheimer's disease based on the determination of the levels of different metabolites, including amino acids, proteins and lipids. The invention also provides a method for diagnosing Alzheimer's disease or mild cognitive impairment in a subject based on the determination of the above metabolites.

## Description

### FIELD OF THE INVENTION

The present invention falls within the field of diagnosis and, more specifically, it relates to the diagnosis of Alzheimer's disease and mild cognitive impairment based on the determination of the serum levels of different proteins, lipids and amino acids.

### BACKGROUND OF THE INVENTION

Alzheimer's disease (AD) is a progressive degenerative disease of the central nervous system characterized by progressive and increasing memory loss, followed by loss of control of limbs and bodily functions and eventual death. It is by far the most common cause of dementia affecting 1 to 6% of people over the age of 65 years and between 10 to 20% of those over 80.

AD is distinguished from other types of dementia by several pathological features, including the progressive appearance in the brain of the patients of senile plaques in the extracellular space between neurons. The plaques have central cores of amyloid deposits formed mainly by fibrils of a 40-42 amino acids peptide referred to amyloid β peptide (Aβ) surrounded by degenerated neuritis and glial cells. This peptide results from the proteolytic processing of a precursor protein called β amyloid precursor protein (βAPP). AD can be classified according to the age of appearance as early onset (age under 60 years) and late onset (age above 60 years), according to the existence of an autosomic dominant inheritance, as familiar AD or sporadic AD. Early onset familiar forms of AD can be associated to known mutation in the genes coding for βAPP, presenilin 1 and presenilin 2 (located, respectively, on chromosomes 21, 14 and 1). These classifications are not mutually exclusive. The most frequent forms are sporadic late-onset forms.

In clinical praxis, diagnosis of AD is carried out using clinical criteria based on the presence of typical clinical hallmarks and the exclusion of other types of dementia using neuroimaging techniques and blood analysis. Using these criteria, diagnostic reliability is acceptable although, according to studies done using brain autopsy, between 10-20% of the patients diagnosed with AD suffered from a different disease. Moreover, the current diagnostic methods can only be carried out when the neurodegenerative process is so advanced that the patient suffers from severe dementia and the brain damages are so extensive that the number of therapeutic measures is limited. Definitive diagnosis requires pathologic examination of post-mortem brain tissue.

Therefore, there is a need for identifying new biomarkers for the diagnosis of AD which are sensitive and specific and which allow distinguishing cognitive impairment due to age from those associated with the early symptoms of the process, as well as to distinguish changes due to AD and due to other degenerative conditions.

Methods are known in the prior art to diagnose AD by detecting the levels of biomarkers present in the brain or cerebrospinal fluid (CSF) of patients. Different biomarkers have been characterised whose determination is carried out in CSF. CSF reflects directly the composition of the extracellular space of the central nervous system and thus, provides higher concentrations as biomarkers. However, CSF can only be retrieved by means of lumbar punction, which is not a routine diagnostic method easily accepted by patients suffering from dementia, let alone in patients with memory disorders. Thus, there is a need for AD biomarkers which can be detected in samples which can be non-invasively retrieved from the body.

Suitable AD biomarkers described in the prior art and which can be detected in plasma include (i) markers derived from the amyloid plaque, (ii) autoantibodies against Aβ o βAPP, (iii) inflammatory markers such IL-6, its receptor or gp130, C-reactive protein or oxidative stress (isoprostanes), (iv) markers of lipidic metabolism (apoE, oxysterols) and (v) vascular disease markers (homocysteine, lipoprotein b C1q) (Scheuner D. et al., Nature Med, 1996, 2, 864-870).

WO08021515A2 describes diagnostic methods for AD and MCI based on the determination of the levels of several free amino acids or dipeptides in a fluid sample of a subject, such as plasma, urine or CSF. The free amino acids or dipeptides that can be used according to the disclosed diagnostic method are an imidazole-containing free amino acid or dipeptide having antioxidant properties, an aromatic-containing free amino acid that is a neurotransmitter, a free amino acid or dipeptide associated with urea metabolism or detoxification and NO formation, a glutamate-derived free amino acid or dipeptide, and an asparate or serine-derived free amino acid.

Moreover, the clinical discrimination between incipient AD and normal cognitive impairment due to aging or cognitive impairment associated to other forms of dementia at prodromal phases is still challenging. Mild cognitive impairment (MCI) is a heterogeneous entity which encompasses AD, frontotemporal dementia (FTD), vascular dementia, Lewy body dementia (LBD), etc. Additionally, not all MCI patients, not even all MCI patients with memory impairment (amnestic MCI) develop Alzheimer's disease. For the above reasons, biomarkers capable of predicting if a patient with MCI is at high risk of developing AD are highly desirable.

Different biomarkers for prediction of the onset and development of AD in a subject have been developed in the art. It has been described that RNA plays an important role in a number of neurodegenerative diseases, and a theoretical framework has been developed to analyze ribonucleoprotein interactions linked to diseases such as Alzheimer's disease (Cirillo D. et al., RNA, 2013 19: 129-140). Combination of Aβ42 concentrations and hippocampal volumes has been suggested to best predict mild MCI progression to AD (Prestia A. et al., Alzheimers Dement., 2013, 9: 677-86).

Despite the efforts made to date, there still exists a long-felt and continuing need in the art for reliable methods useful for diagnosing both Alzheimer's disease and MCI based on the analysis of bodily fluids, which can be easily obtainable by non-invasive means and for reliable methods for predicting the onset of AD in patients with MCI.

### SUMMARY OF THE INVENTION

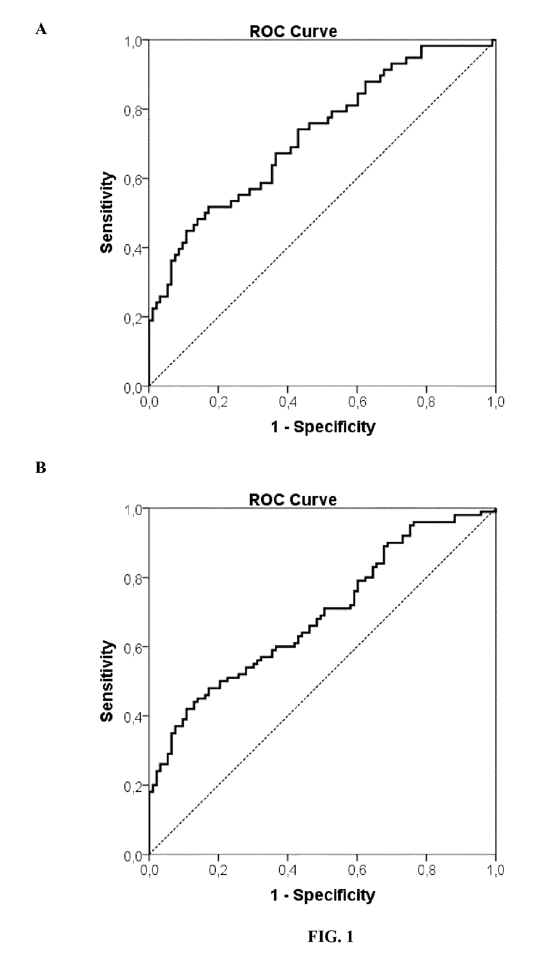

The inventors of the present invention have observed that the determination of the levels of different biomarkers allow the identification of patients with Alzheimer's disease and/or amnestic mild cognitive impairment. The panel of biomarkers identified by the inventors, which include different types of metabolites like proteins, amino acids and lipids, is useful for diagnosing Alzheimer's disease and/or mild cognitive impairment and for identifying a patient with mild cognitive impairment which is at high risk of developing Alzheimer's disease. In particular, as illustrated by the examples of the application, the inventors have shown that the levels of the biomarkers 21:0 ceramide and apolipoprotein C-I (two-variable model) are able to discriminate Alzheimer's disease and MCI patients from healthy controls with an AUC of 0.689 and 0.724 respectively (figure 1 and table 9). The discrimination power of the model can be significantly improved by increasing the number of biomarkers of the signature, reaching AUC values over 0.900 when five or more biomarkers are determined (Table 9).

Based on the previous findings, the following inventive aspects have been developed.

In a first aspect, the invention relates to an *in vitro* method for determining the likelihood that a patient with mild cognitive impairment develops Alzheimer's disease comprising
(a) determining in a sample from said patient the level of at least one biomarker selected from the biomarkers of Table 1, and
(b) comparing the level of said at least one biomarker with its reference value,
wherein decreased levels of at least one biomarker selected from the group consisting of 21:0 ceramide, apolipoprotein C-I, sarcosine, conjugated linoleic acid, docosahexaenoic acid, apolipoprotein C-II and glutamic acid compared to its reference value and/or increased levels of at least one biomarker selected from the group consisting of alanine and cortisol compared to its reference value are indicative of a high likelihood of the subject developing Alzheimer's disease.

In a second aspect, the invention relates to an *in vitro* method for the diagnosis of Alzheimer's disease or mild cognitive impairment in a subject comprising:
(a) determining in a sample from said subject the level of at least one biomarker selected from the biomarkers of Table 2, and
(b) comparing the level of said at least one biomarker with its reference value,
wherein decreased levels of at least one biomarker selected from the group consisting of 21:0 ceramide, apolipoprotein C-I, sarcosine, conjugated linoleic acid, docosahexaenoic acid and apolipoprotein C-II compared to its reference value and/or increased levels of the biomarker alanine compared to its reference value are indicative of the subject suffering from Alzheimer's disease or mild cognitive impairment.

In a third aspect, the invention relates to a method for the treatment and/or prevention of Alzheimer's disease comprising administering a therapy for the treatment of Alzheimer's disease to a patient with mild cognitive impairment, wherein the patient is selected for said treatment if a sample from said patient contains decreased levels of at least one biomarker selected from the group consisting of 21:0 ceramide, apolipoprotein C-I, sarcosine, conjugated linoleic acid, docosahexaenoic acid, apolipoprotein C-II and glutamic acid compared to its reference value and/or contains increased levels of at least one biomarker selected from the group consisting of alanine and cortisol compared to its reference value.

### BRIEF DESCRIPTION OF THE FIGURES

Figure 1. ROC Curve obtained from Cer(d18:1/21:0) and APO C-I variables into aMCI vs. Healthy Control (A) and Alzheimer vs. Healthy Control (B) comparisons.
Figure 2. ROC Curve obtained from Cer(d18:1/21:0), APO C-I and Glutamic acid variables into aMCI vs. Healthy Control (A) and Alzheimer vs. Healthy Control (B) comparisons.
Figure 3. ROC Curve obtained from Cer(d18:1/21:0), APO C-I and Alanine variables into aMCI vs. Healthy Control (A) and Alzheimer vs. Healthy Control (B) comparisons.
Figure 4. ROC Curve obtained from Cer(d18:1/21:0), APO C-I and DHA variables into aMCI vs. Healthy Control (A) and Alzheimer vs. Healthy Control (B) comparisons.
Figure 5. ROC Curve obtained from Cer(d18:1/21:0), APO C-I and CLA variables into aMCI vs. Healthy Control (A) and Alzheimer vs. Healthy Control (B) comparisons.
Figure 6. ROC Curve obtained from Cer(d18:1/21:0), APO C-I and Sarcosine variables into aMCI vs. Healthy Control (A) and Alzheimer vs. Healthy Control (B) comparisons.
Figure 7. ROC Curve obtained from Cer(d18:1/21:0), APO C-I and APO C-II variables into aMCI vs. Healthy Control (A) and Alzheimer vs. Healthy Control (B) comparisons.
Figure 8. ROC Curve obtained from Cer(d18:1/21:0), APO C-I and APO E cod variables into aMCI vs. Healthy Control (A) and Alzheimer vs. Healthy Control (B) comparisons.
Figure 9. ROC Curve obtained from Cer(d18:1/21:0), APO C-I and Cortisol variables into aMCI vs. Healthy Control (A) and Alzheimer vs. Healthy Control (B) comparisons.
Figure 10. ROC Curve obtained from Cer(d18:1/21:0), APO C-I, APO C-II and Cortisol variables into aMCI vs. Healthy Control (A) and Alzheimer vs. Healthy Control (B) comparisons.
Figure 11. ROC Curve obtained from Cer(d18:1/21:0), APO C-I, Cortisol and Glutamic acid variables into aMCI vs. Healthy Control (A) and Alzheimer vs. Healthy Control (B) comparisons.
Figure 12. ROC Curve obtained from Cer(d18:1/21:0), APO C-I, Cortisol and Alanine variables into aMCI vs. Healthy Control (A) and Alzheimer vs. Healthy Control (B) comparisons.
Figure 13. ROC Curve obtained from Cer(d18:1/21:0), APO C-I, Cortisol, Alanine and Glutamic acid variables into aMCI vs. Healthy Control (A) and Alzheimer vs. Healthy Control (B) comparisons.
Figure 14. ROC Curve obtained from Cer(d18:1/21:0), APO C-I, Cortisol, Alanine, Glutamic acid and APO C-II variables into aMCI vs. Healthy Control (A) and Alzheimer vs. Healthy Control (B) comparisons.
Figure 15. ROC Curve obtained from Cer(d18:1/21:0), APO C-I, Cortisol, Alanine, Glutamic acid APO C-II and CLA variables into aMCI vs. Healthy Control (A) and Alzheimer vs. Healthy Control (B) comparisons.
Figure 16. ROC Curve obtained from Cer(d18:1/21:0), APO C-I, Cortisol, Alanine, Glutamic acid APO C-II, CLA and DHA variables into aMCI vs. Healthy Control (A) and Alzheimer vs. Healthy Control (B) comparisons.
Figure 17. ROC Curve obtained from Cer(d18:1/21:0), APO C-I, Cortisol, Alanine, Glutamic acid APO C-II, CLA, DHA and APO E cod variables into aMCI vs. Healthy Control (A) and Alzheimer vs. Healthy Control (B) comparisons.

### DETAILED DESCRIPTION OF THE INVENTION

### Method for determining the likelihood that a patient with mild cognitive impairment develops Alzheimer's disease

In a first aspect, the invention relates to an *in vitro* method, hereinafter first method of the invention, for determining the likelihood that a patient with mild cognitive impairment develops Alzheimer's disease comprising
(a) determining in a sample from said patient the level of at least one biomarker selected from the biomarkers of Table 1, and
(b) comparing the level of said at least one biomarker with its reference value,
wherein decreased levels of at least one biomarker selected from the group consisting of 21:0 ceramide, apolipoprotein C-I, sarcosine, conjugated linoleic acid, docosahexaenoic acid, apolipoprotein C-II and glutamic acid compared to its reference value and/or increased levels of at least one biomarker selected from the group consisting of alanine and cortisol compared to its reference value are indicative of a high likelihood of the subject developing Alzheimer's disease.

The term "Alzheimer's disease" or "AD", as used herein, refers a mental deterioration associated with specific degenerative brain disease that is characterized by senile plaques, neuritic tangles and progressive neuronal loss which manifests clinically in progressive memory deficits, confusion, behavioral problems, inability to care for oneself, gradual physical deterioration and, ultimately, death. Patients suffering Alzheimer's disease are identified using the NINCDS-ADRDA (National Institute of Neurological and Communicative Disorders and the Alzheimer's Disease and Related Disorders Association) criteria: Clinical Dementia Rating (CDR) = 1; Mini Mental State Examination (MMSE) between 16 and 24 points and Medial temporal atrophy (determined by Magnetic Resonance Imaging, MRI) >3 points in Scheltens scale.

The term "Alzheimer's disease", as used herein, is intended to include all the stages of the disease, including the following stages defined by NINCDS-ADRDA Alzheimer's Criteria for diagnosis in 1984:
- Definite Alzheimer's disease: The patient meets the criteria for probable Alzheimer's disease and has histopathologic evidence of AD via autopsy or biopsy.
- Probable or prodromal Alzheimer's disease: Dementia has been established by clinical and neuropsychological examination. Cognitive impairments also have to be progressive and be present in two or more areas of cognition. The onset of the deficits has been between the ages of 40 and 90 years and finally there must be an absence of other diseases capable of producing a dementia syndrome.
- Possible or non-prodromal Alzheimer's disease: There is a dementia syndrome with an atypical onset, presentation or progression; and without a known etiology; but no co-morbid diseases capable of producing dementia are believed to be in the origin of it.

In a preferred embodiment of the first method of the invention, the Alzheimer's disease is prodromal Alzheimer's disease.

The term "prodromal Alzheimer's disease" or "MCI with probable Alzheimer's disease" refers to patients showing MCI and which are considered as showing high risk for conversion to Alzheimer's disease. Criteria for identifying a patient as probable AD are those as defined by the NINCDS-ADRDA criteria (McKhann G. et al., Neurology 1984, 34: 939-44), namely, dementia established by clinical and neuropsychological examination, progressive cognitive impairment present in two or more areas of cognition, onset of the deficits between the ages of 40 and 90 years and absence of other diseases capable of producing a dementia syndrome.

The term "mild cognitive impairment" or MCI is related to a transitional stage of cognitive impairment between normal aging and early Alzheimer's disease. Patients are usually identified as having MCI if they fulfill the Mayo Clinic criteria (Clinical Dementia Rating, CDR = 0.5, they show a medial temporal atrophy (determined by Magnetic Resonance Imaging, MRI) which is higher than 3 points in Scheltens scale, they show a pattern of parietal and/or temporal hypometabolism in Positron Emission Tomography with 18-fluorodeoxyglucose (PET-FDG) (suggestive of AD).

In a particular embodiment, the MCI is amnestic MCI. The term "amnestic MCI" or "aMCI", as used herein, refers to a type of MCI, the predominant symptom of which is memory loss. This term has been defined in Petersen et. al. Arch Neurol. 1999 Mar;56(3):303-8.

The term "*in vitro*", as used herein, means that it is not performed over the human or animal body but in a sample isolated from the body.

The term "patient", as used herein, refers to all animals classified as mammals and includes, but is not restricted to, domestic and farm animals, primates and humans, e.g., human beings, non-human primates, cows, horses, pigs, sheep, goats, dogs, cats, or rodents. Preferably, the patient is a male or female human of any age or race. In the context of the first method of the invention, the subject suffers from MCI.

The term "patient with mild cognitive impairment", as used herein, refers to patients that have been diagnosed with MCI. In a preferred embodiment of the first method of the invention, the patient is a patient with amnestic MCI.

The term "method for determining the likelihood", as used herein, refers to a method for determining the probability of a particular event. In the context of the first method of the invention, determining the likelihood of a patient with MCI developing Alzheimer's disease refers to determining whether said patient has a high likelihood of developing Alzheimer's disease. The term "high likelihood", as used herein, refers to a significant probability of developing Alzheimer's disease. In a particular embodiment, a high likelihood is at least about 20%, including but not limited to about 25%, 30%, 35%, 40%, 45%, 50%, 55%, 60%, 65%, 70%, 75%, 80%, 85%, 90%, 95%, 100%, 150%, 200%, 300%, 400%, 500%, 600%, 700%, 800%, 900%, 1000%, and 1500%. In one particular embodiment, a high likelihood is at least 100%. In other embodiments, a high likelihood is at least 200%, at least 300%, at least 400%, at least 500%, at least 700%, at least 800%, at least 900% and at least 1000%. Other cut-offs or ranges as deemed suitable by the person skilled in the art to characterize the invention are however also contemplated, and those are also within scope of the present invention.

The first step of the first method of the invention comprises the determination of the levels of at least one biomarker selected from the biomarkers of Table 1 in a sample from a patient with MCI.

**Table 1**

| **BIOMARKER** |
|---|
| 21:0 ceramide |
| Apolipoprotein C-I (Apo C-I) |
| Alanine |
| Sarcosine |
| Conjugated linoleic acid (CLA) |
| Docohexaenoic acid (DHA) |
| Apolipoprotein C-II (Apo C-II) |
| Glutamic acid |
| Cortisol |

The term "sample", as used herewith, refers to any sample collected from a subject, in which the markers of the methods of the invention can be measured. Suitable samples for use in the present invention include any bodily fluid.

In a preferred embodiment, the sample is a bodily fluid sample. The term "bodily fluid sample", as used herewith, refers to a sample of a liquid derived from the body of a living organism. Illustrative, non-limitative, examples of bodily fluids include tears, blood, plasma or blood serum. Preferably, the bodily fluid sample is blood, plasma or blood serum.

The term "biomarker", as used herewith, refers to a biomolecule, such as a protein, a nucleic acid, a lipid or a carbohydrate, the occurrence or amount of which is characteristic for a specific situation, for example, a disease such as Alzheimer's disease or MCI.

The biomarkers useful for the method of diagnosis of the invention are those included in Table 1.

The term "21:0 ceramide", or "ceramide do 8:1/21:0", or "N-(beneicosanoyl)-sphing-4-enine-1-phosphocholine" as used herein, refers to a sphingolipid of formula:

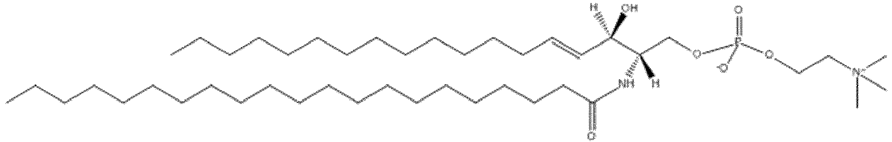

The term "apolipoprotein C-I" or "Apo C-I", as used herein, refers to a protein normally found in plasma and responsible for the activation of esterified lecithin cholesterol with an important role in the exchange of esterified cholesterol between lipoproteins and in removal of cholesterol from tissues. In humans, Apo C-I is encoded by the gene *APOCl.* The Apo C-I can be from any origin, for example human, bovine, murine, equine, canine, etc., depending on the subject which is going to be diagnosed with the first method of the invention. In a particular embodiment, the Apo C-I is the human protein with the UniProt accession number P02654 (release of March 19, 2014).

The term "alanine", abbreviated "Ala" or "A", as used herein, refers to the α-amino acid of formula:

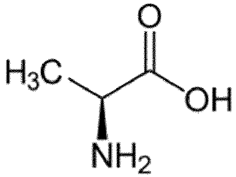

The term "alanine", as used herein, refers to the enantiomer L-alanine.

The term "sarcosine" or "N-methylglycine", as used herein, refers to the amino acid of formula:

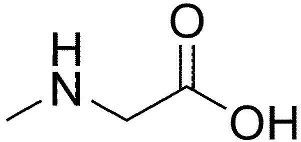

The term "conjugated linoleic acid" or "CLA" refers to a family of isomers of linoleic acid. The term "linoleic acid" or "octadecadienoic fatty acid", as used herein, refers to an unsaturated omega-6 fatty acid with 18 carbon atoms and two *cis* double bonds at positions 9 and 12. The linoleic acid has the formula:

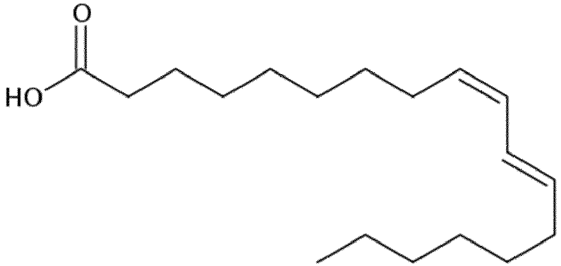

The term "CLA", as used herein, it is intended to encompass all positional and geometric isomers of linoleic acid with two conjugated carbon-carbon double bonds any place in molecule. The term "conjugated double bonds" means that the double bonds are separated by a single bond between those two double bonds. Examples of CLA include *cis* and *trans* isomers ("E/Z isomers") of the following positional isomers: 2,4-octadecadienoic acid; 4,6-octadecadienoic acid; 6,8-octadecadienoic acid; 7,9-octadecadienoic acid; 8,10-octadecadienoic acid; 9,11-octadecadienoic acid; 10,12 octadecadienoic acid; and 11,13-octadecadienoic acid. As used herein, "CLA" encompasses a single isomer, a selected mixture of two or more isomers, and a non-selected mixture of isomers obtained from natural sources, as well as synthetic and semisynthetic CLA.

The term "DHA" or "docohexaenoic acid" or "cervonic acid", as used herein, refers to an omega-3 fatty acid of formula:

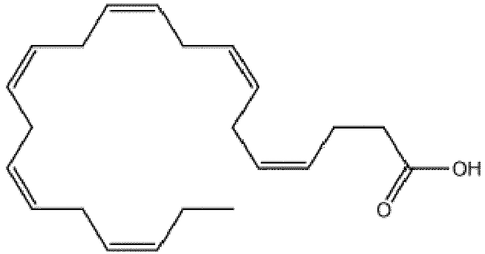

In the nomenclature of fatty acids its short name is 22:6(n-3).

The term "apolipoprotein C-II" or "Apo C-II", as used herein, refers to a protein normally found in plasma where it is a component of very low density lipoproteins (VLDL) and chylomicrons. This protein is responsible for the activation of the enzyme lipoprotein lipase in capillaries, which hydrolyzes triglycerides and thus provides free fatty acids for cells. In humans, Apo C-II is encoded by the gene *APOC2.* The Apo C-II can be from any origin, for example human, bovine, murine, equine, canine, etc., depending on the subject which is going to be diagnosed with the first method of the invention. In a particular embodiment, the Apo C-II is the human protein with the UniProt accession number P02655 (March 19, 2014).

The term "glutamic acid", abbreviated as "Glu" or "E", as used herein, refers to the α-amino acid of formula:

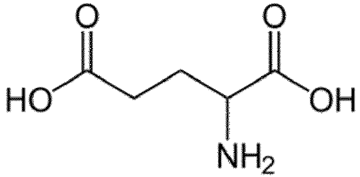

The term "glutamic acid", as used herein, refers to the enantiomer L-glutamic acid.

The term "cortisol" or "hydrocortisone", as used herein, refers to a glucocorticoid of formula:

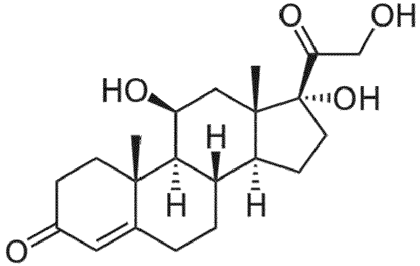

In a particular embodiment, the first method of the invention comprises determining at least two biomarkers selected from the biomarkers of Table 1. In a preferred embodiment, said two biomarkers are 21:0 ceramide and a biomarker selected from apolipoprotein C-I, alanine, sarcosine, CLA, DHA, apolipoprotein C-II, glutamic acid and cortisol. In a more preferred embodiment, said two biomarkers are 21:0 ceramide and apolipoprotein C-II.

In another particular embodiment, the first method of the invention comprises determining at least three biomarkers selected from the biomarkers of Table 1. In a preferred embodiment, said three biomarkers are 21:0 ceramide, apolipoprotein C-I and one biomarker selected from alanine, sarcosine, CLA, DHA, apolipoprotein C-II, glutamic acid and cortisol. In a particular embodiment, said three biomarkers are:
(i) 21:0 ceramide, apolipoprotein C-I and glutamic acid,
(ii) 21:0 ceramide, apolipoprotein C-I and alanine,
(iii) 21:0 ceramide, apolipoprotein C-I and cortisol,
(iv) 21:0 ceramide, apolipoprotein C-I and docosahexaenoic acid,
(v) 21:0 ceramide, apolipoprotein C-I and conjugated linoleic acid,
(vi) 21:0 ceramide, apolipoprotein C-I and sarcosine,
(vii) 21:0 ceramide, apolipoprotein C-I and apolipoprotein C-II.

In another particular embodiment, the first method of the invention comprises determining at least four biomarkers selected from the biomarkers of Table 1. In a preferred embodiment, said four biomarkers are 21:0 ceramide, apolipoprotein C-I and two biomarkers selected from alanine, sarcosine, CLA, DHA, apolipoprotein C-II, glutamic acid and cortisol. In a particular embodiment, said four biomarkers are 21:0 ceramide, apolipoprotein C-I, cortisol and one biomarker selected from alanine, sarcosine, CLA, DHA, apolipoprotein C-II and glutamic acid. In a more particular embodiment, said four biomarkers are:
(i) 21:0 ceramide, apolipoprotein C-I, apolipoprotein C-II and cortisol,
(ii) 21:0 ceramide, apolipoprotein C-I, cortisol and glutamic acid,
(iii)21:0 ceramide, apolipoprotein C-I, cortisol and alanine.

In another particular embodiment, the first method of the invention comprises determining at least five biomarkers selected from the biomarkers of Table 1. In a preferred embodiment, said five biomarkers are 21:0 ceramide, apolipoprotein C-I and three biomarkers selected from alanine, sarcosine, CLA, DHA, apolipoprotein C-II, glutamic acid and cortisol. In a particular embodiment, said five biomarkers are 21:0 ceramide, apolipoprotein C-I, cortisol and two biomarkers selected from alanine, sarcosine, CLA, DHA, apolipoprotein C-II and glutamic acid. In a more particular embodiment, said five biomarkers are 21:0 ceramide, apolipoprotein C-I, cortisol, alanine and one biomarker selected from sarcosine, CLA, DHA, apolipoprotein C-II and glutamic acid. In another more particular embodiment, said five biomarkers are 21:0 ceramide, apolipoprotein C-I, cortisol, glutamic acid and one biomarker selected from alanine, sarcosine, CLA, DHA and apolipoprotein C-II. In an even more particular embodiment, said five biomarkers are 21:0 ceramide, apolipoprotein C-I, cortisol, alanine and glutamic acid.

In another particular embodiment, the first method of the invention comprises determining at least six biomarkers selected from the biomarkers of Table 1. In a preferred embodiment, said six biomarkers are 21:0 ceramide, apolipoprotein C-I and four biomarkers selected from alanine, sarcosine, CLA, DHA, apolipoprotein C-II, glutamic acid and cortisol. In a particular embodiment, said six biomarkers are 21:0 ceramide, apolipoprotein C-I, cortisol and three biomarkers selected from alanine, sarcosine, CLA, DHA, apolipoprotein C-II and glutamic acid. In a more particular embodiment, said six biomarkers are 21:0 ceramide, apolipoprotein C-I, cortisol, alanine and two biomarkers selected from sarcosine, CLA, DHA, apolipoprotein C-II and glutamic acid. In a more particular embodiment, said six biomarkers are 21:0 ceramide, apolipoprotein C-I, cortisol, alanine, glutamic acid and one biomarker selected from sarcosine, CLA, DHA and apolipoprotein C-II. In an even more particular embodiment, said six biomarkers are 21:0 ceramide, apolipoprotein C-I, cortisol, alanine, glutamic acid and apolipoprotein C-II.

In another particular embodiment, the first method of the invention comprises determining at least seven biomarkers selected from the biomarkers of Table 1. In a preferred embodiment, said seven biomarkers are 21:0 ceramide, apolipoprotein C-I and five biomarkers selected from alanine, sarcosine, CLA, DHA, apolipoprotein C-II, glutamic acid and cortisol. In a particular embodiment, said seven biomarkers are 21:0 ceramide, apolipoprotein C-I, cortisol and four biomarkers selected from alanine, sarcosine, CLA, DHA, apolipoprotein C-II and glutamic acid. In a more particular embodiment, said seven biomarkers are 21:0 ceramide, apolipoprotein C-I, cortisol, alanine and three biomarkers selected from sarcosine, CLA, DHA, apolipoprotein C-II and glutamic acid. In a more particular embodiment, said seven biomarkers are 21:0 ceramide, apolipoprotein C-I, cortisol, alanine, glutamic acid and two biomarkers selected from sarcosine, CLA, DHA and apolipoprotein C-II. In a more particular embodiment, said seven biomarkers are 21:0 ceramide, apolipoprotein C-I, cortisol, alanine, glutamic acid, apolipoprotein C-II and one biomarker selected from sarcosine, CLA and DHA. In an even more particular embodiment, said seven biomarkers are 21:0 ceramide, apolipoprotein C-I, cortisol, alanine, glutamic acid, apolipoprotein C-II and CLA.

In another particular embodiment, the first method of the invention comprises determining at least eight biomarkers selected from the biomarkers of Table 1. In a preferred embodiment, said eight biomarkers are 21:0 ceramide, apolipoprotein C-I and six biomarkers selected from alanine, sarcosine, CLA, DHA, apolipoprotein C-II, glutamic acid and cortisol. In a particular embodiment, said eight biomarkers are 21:0 ceramide, apolipoprotein C-I, cortisol and five biomarkers selected from alanine, sarcosine, CLA, DHA, apolipoprotein C-II and glutamic acid. In a more particular embodiment, said eight biomarkers are 21:0 ceramide, apolipoprotein C-I, cortisol, alanine and four biomarkers selected from sarcosine, CLA, DHA, apolipoprotein C-II and glutamic acid. In a more particular embodiment, said eight biomarkers are 21:0 ceramide, apolipoprotein C-I, cortisol, alanine, glutamic acid and three biomarkers selected from sarcosine, CLA, DHA and apolipoprotein C-II. In a more particular embodiment, said eight biomarkers are 21:0 ceramide, apolipoprotein C-I, cortisol, alanine, glutamic acid, apolipoprotein C-II and two biomarkers selected from sarcosine, CLA and DHA. In a more particular embodiment, said eight biomarkers are 21:0 ceramide, apolipoprotein C-I, cortisol, alanine, glutamic acid, apolipoprotein C-II, CLA and one biomarker selected from sarcosine and DHA. In an even more particular embodiment, said eight biomarkers are 21:0 ceramide, apolipoprotein C-I, cortisol, alanine, glutamic acid, apolipoprotein C-II, CLA and DHA.

In another particular embodiment, the first method of the invention comprises determining all the biomarkers from Table 1.

In a particular embodiment, when the first method of the invention comprises determining at least one, at least two, at least three, at least four, at least five, at least six, at least seven or at least eight biomarkers, any additional biomarker is not selected from the biomarkers of Table 1.

The first method of the invention involves the determination of the level of the biomarkers mentioned above. The term "level", as used herein, refers to the quantity of a biomarker detectable in a sample. The methods for determining the level of the biomarkers according to the first method of the invention will depend on the type of biomarker, namely, lipid biomarkers, protein biomarkers or amino acid biomarkers.

The level of lipid biomarkers according to the first method of the invention, namely 21:0 ceramide, CLA, DHA and cortisol, can be determined by any method known in the art suitable for the determination and quantification of a lipid in a sample. By way of a non-limiting illustration, the level of a particular lipid can be determined by means of chromatography, mass spectrometry, nuclear resonance spectroscopy, fluorescence spectroscopy or dual polarization interferometry, a high performance separation method such as HPLC and/or an immunological method. In a particular embodiment, when the biomarker according to the first method of the invention is a lipid, the levels of said biomarker are determined by means of a separation technique coupled to a method for identification and quantification of the lipid biomarker. In a more particular embodiment, the separation technique is a chromatography, preferably HPLC, more preferably UPLC^{®}, and the method for identification and quantification of the lipid biomarker is mass spectrometry, preferably MS-TOF.

In an even more particular embodiment, when the biomarker is 21:0 ceramide, CLA or DHA, the levels of said biomarker are determined by means of a separation technique, preferably chromatography, more preferably HPLC, even more preferably UPLC^{®}, coupled to a method for identification and quantification of the lipid biomarker, preferably mass spectrometry, more preferably MS-TOF.

The term "HPLC" or "high performance liquid chromatography" as used herein, refers to a technique used in analytic chemistry to separate, identify and quantify the components of a mixture in which the degree of separation is increased by forcing a mobile phase under pressure through a stationary phase on a support matrix, typically a densely packed column. In a particular embodiment, the HPLC used for the separation of the lipid biomarkers is "UPLC^{®}" or "Ultra Performance Liquid Chromatography^{®}", which refers to a HPLC system improved to work at pressures up to 100 MPa, which are much higher than the pressures used in standard HPLC, and which therefore allow using much smaller particle sizes in the columns.

The term "MS" or "mass-spectrometry", as used herein, refers to various methods such as tandem mass spectrometry, matrix assisted laser desorption ionization (MALDI), time-of-flight (TOF) mass spectrometry, MALDI-TOF-TOF mass spectrometry, MALDI Quadrupole-time-of flight (Q-TOF) mass spectrometry, electrospray ionization (ESI)-TOF mass spectrometry, ESI-Q-TOF, ESI-TOF-TOF, ESI-ion trap mass spectrometry, ESI Triple quadrupole mass spectrometry, ESI Fourier Transform mass spectrometry (FTMS), MALDI-FTMS, MALDI-Ion Trap-TOF, and ESI-Ion Trap TOF. These mass spectrometry methods are well known in the art. At its most basic level, mass spectrometry involves ionizing a molecule and then measuring the mass of the resulting ion. Since molecules ionize in a way that is well known, the molecular weight of the molecule can generally be accurately determined from the mass of the ion. Tandem mass spectrometry, for instance, may be used to identify proteins because it can provide information in addition to parent ion molecular weight. Tandem mass spectrometry involves first obtaining a mass spectrum of the ion of interest, then fragmenting that ion and obtaining a mass spectrum of the fragments. Tandem mass spectrometry thus provides both molecular weight information and a fragmentation pattern that can be used in combination along with the molecular weight information to identify the exact sequence of a peptide or protein.

The term "MS-TOF" or "time-of-flight mass spectrometry" as used herein, refers to a method of mass spectrometry in which an ion's mass-to-charge ratio is determined via a time measurement. Ions are accelerated by an electric field of known strength. This acceleration results in an ion having the same kinetic energy as any other ion that has the same charge. The velocity of the ion depends on the mass-to-charge ratio. The time that it subsequently takes for the particle to reach a detector at a known distance, which will depend on the mass-to-charge ratio of the particle (heavier particles reach lower speeds), is measured.

In a particular embodiment, when the biomarker is cortisol, the levels of said biomarker can be determined by an immunological method, preferably a chemiluminiscent enzyme immunoassay, more preferably a solid-phase chemiluminiscent enzyme immunoassay

As used herein, "immunological method", when applied to a determination, relates to any method which involves the use of one or more antibodies specific for a target substance in order to determine the amount/concentration of said target substance excluding other substances found in the sample. Suitable immunological methods include, without limitation, Western blot, immunoprecipitation, enzyme-linked immunosorbent assay (ELISA), inmunoturbidimetry, surface plasmon resonance, radioimmunoassay (RIA) and chemiluminiscent enzyme immunoassay.

The term "chemiluminiscent enzyme immunoassay", as used herein, refers to an immunological method in which a specific antibody selectively binding to the target substance is immobilized to a surface, and the target substance is selectively taken out from a liquid layer. Such a method in which a reaction is carried out in a test tube, microplate or the like, and the color tone or fluorescent substance or luminescent substance appeared in a liquid layer is measured, is referred to as liquid-phase type assay method. On the other hand, solid phase type immunoassay methods are the methods of measuring the target substance by intensively capturing an immune complex of the target substance and labeled antibody (labeled with a visualizing substance such as gold colloid particle and color latex) in a section linearly arranged with antibodies on a test device, and visually or optically reading the captured immune complex.

The level of amino acid biomarkers according to the first method of the invention, namely alanine, glutamic acid and sarcosine, can be determined by any method known in the art suitable for the determination and quantification of an amino acid in a sample.

Analytical methods for amino acids include methods including a derivatization step. During derivatization, the amino acid is reacted with a derivatizing reagent that facilitates analysis of amino acids in the sample. Derivatizing agents typically react with the free amino groups of amino acids in the sample. Common reagents for derivatizing amino acids include isothiocyanates (e.g., phenyl isothiocynate (PITC)), o-phthaldialdehyde (OPA), 2,4- dinitrofluorobenzene (DNFB), and Nα-(2,4-dinitro-5-fluorophenyl)-L-alainamide (FDAA). Derivatizing agents are useful because they may include substituents that facilitate analysis of the derivatized amino acid. For example, derivatizing agents may include chromophores for UV-absorption detection or fluorophores for fluorescent detection.

Derivatized amino acids may be separated and detected by performing chromatography such as liquid chromatography (LC) or gas chromatography (GC), coupled with mass spectrometry (i.e., LC-MS or GC-MS).

In a particular embodiment, when the biomarker according to the first method of the invention is an amino acid, the levels of said biomarker are determined by means of a separation technique coupled to a method for identification and quantification of the amino acid biomarker. In a more particular embodiment, the separation technique is a chromatography, preferably HPLC, more preferably UPLC^{®}, and the method for identification and quantification of the lipid biomarker is mass spectrometry, preferably MS-SQD.

In an even more particular embodiment, when the biomarker is glutamic acid, alanine or sarcosine, the levels of said biomarker are determined by means of a separation technique, preferably chromatography, more preferably HPLC, even more preferably UPLC^{®}, coupled to a method for identification and quantification of the lipid biomarker, preferably mass spectrometry, more preferably MS-SQD.

The terms "HPLC", "UPLC" and "mass spectrometry" have been previously defined.

The term "MS-SQD" or "single quadrupole mass spectrometry" as used herein, refers to a method of mass spectrometry in which a quadrupole, consisting on four cylindrical parallel metal rods, is used for filtering sample ions bases on their mass-to-charge ratio (*m*/*z*). Ions are separated in a quadrupole based on the stability of their trajectories in the oscillating electric fields that are applied to the rods.

The level of protein biomarkers according to the first method of the invention, namely Apo C-I and Apo C-II, can be determined by any method known in the art suitable for the determination and quantification of a protein in a sample. By way of a non-limiting illustration, the level of a protein can be determined by means of a technique which comprises the use of antibodies with the capacity for binding specifically to the assayed protein (or to fragments thereof containing the antigenic determinants) and subsequent quantification of the resulting antigen-antibody complexes, or alternatively by means of a technique which does not comprise the use of antibodies such as, for example, by techniques based on mass spectroscopy. The antibodies can be monoclonal, polyclonal or fragment thereof, Fv, Fab, Fab' and F(ab')2, scFv, diabodies, triabodies, tetrabodies and humanized antibodies. Similarly, the antibodies may be labeled. Illustrative, but non-exclusive, examples of markers that can be herein used include radioactive isotopes, enzymes, fluorophores, chemoluminescent reagents, enzyme cofactors or substrates, enzyme inhibitors, particles, or dyes. There is a wide variety of known test that can be used according to the present invention, such as combined application of non-labeled antibodies (primary antibodies) and labeled antibodies (secondary antibodies), Western blot or immunoblot, ELISA (enzyme-linked immunosorbent assay), RIA (radioimmunoassay), competitive EIA (enzyme immunoassay), DAS-ELISA (double antibody sandwich ELISA), two-dimensional gel electrophoresis, capillary electrophoresis, immunocytochemical and immunohistochemical techniques, immunoturbidimetry, immunofluorescence, techniques based on the use of biochips or protein microarrays including specific antibodies or assays based on the colloidal precipitation in formats such as reagent strips and assays based on antibody-linked quantum dots. Other forms of detecting and quantifying proteins include, for instance, affinity chromatography techniques or ligand-binding assays.

In a particular embodiment, when the biomarker according to the first method of the invention is a protein, the levels of said biomarker are determined by an immunological method, preferably and ELISA. In a more particular embodiment, when the biomarker is Apo C-I, the levels of said biomarker are determined by ELISA.

The term "ELISA" or "enzyme-linked immunosorbent assay", as used herein, stands for enzyme-linked immunosorbent assay and relates to an assay by which an unknown amount of target substance (the biomarkers of the first method of the invention) is affixed to a surface, and then a specific antibody is washed over the surface so that it can bind to the antigen. This antibody is linked to an enzyme, and in the final step a substance is added that the enzyme can convert to some detectable signal. Different types of ELISA assays are known and can be applied to the method of the invention, including direct ELISA, sandwich ELISA, competitive ELISA and ELISA reverse method & device (ELISA-R m&d).

The ELISA sandwich assay involves coating a support with a first antibody specific for the biomarker, applying the sample containing the biomarker which will result in the binding of the biomarker to the first antibody and applying a second antibody also specific for the biomarker, wherein said second antibody is usually coupled to a detectable tag or to a substrate-modifying enzyme. The signal generated by the tag or by the converted substrate is the proportional to the amount of antigen in the sample.

In a particular embodiment, the ELISA analysis of Apo C-I is carried out according to the method described in the examples of the present application.

In another particular embodiment, when the biomarker according to the first method of the invention is a protein, the levels of said biomarker are determined by immunoturbidimetry. In a more particular embodiment, when the biomarker is Apo C-II, the levels of said biomarker are determined by immunoturbidimetry.

The term "immunoturbidimetry", as used herein, refers to a technique for the detection of an analyte in a sample based on the reaction of the analyte with an antibody, which leads to the formation of an antibody-antigen immune complex between the analyte and the antibody that precipitates, increasing the turbidity of the sample. As a result, when light is passed through the reaction solution, some light is scattered by the sample, some light is absorbed by the sample and the rest passes through the sample. The amount of absorbed light is directly proportional to the analyte concentration or, in other words, the transmittal light signal is directly proportional to the analyte concentration. Immunoturbidimetric assay kits for the quantification of different proteins, such as Apo C-II, are commercially available. In a particular embodiment, the immunoturbidimetric analysis of Apo C-II is carried out according to the method described in the examples of the present application.

The second step of the first method of the invention comprises comparing the level of the biomarkers with its reference value.

The term "reference value", as used herein, relates to a predetermined criteria used as a reference for evaluating the values or data obtained from the samples collected from a subject. The reference value or reference level can be an absolute value, a relative value, a value that has an upper or a lower limit, a range of values, an average value, a median value, a mean value, or a value as compared to a particular control or baseline value. A reference value can be based on an individual sample value, such as for example, a value obtained from a sample from the subject being tested, but at an earlier point in time. The reference value can be based on a large number of samples, such as from population of subjects of the chronological age matched group, or based on a pool of samples including or excluding the sample to be tested.

The reference value according to the first method of the invention can obtained from one or more subjects who do not suffer from Alzheimer's disease (i.e., control subjects). In a particular embodiment, the reference value is obtained from one or more subjects that do not suffer from MCI either. In another particular embodiment, the reference value is obtained from one or more subjects that suffers from MCI but have not developed Alzheimer's disease.

According to the first method for the invention, a subject is considered that does not suffer from Alzheimer's disease or MCI if said subject does not meet the aforementioned diagnostic criteria for Alzheimer's disease or MCI.

According to the first method of the invention, decreased levels of at least one biomarker selected from the group consisting of C21:0 ceramide, apolipoprotein C-I, sarcosine, conjugated linoleic acid, docosahexaenoic acid, apolipoprotein C-II and glutamic acid compared to its reference value and/or increased levels of at least one biomarker selected from the group consisting of alanine and cortisol compared to its reference value are indicative of a high likelihood of the subject developing Alzheimer's disease.

According to the first method of the invention, the level of a biomarker is considered "decreased" when the level of said biomarker in a sample is lower than a reference value. The levels of a biomarker are considered to be lower than its reference value when it is at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 100%, at least 110%, at least 120%, at least 130%, at least 140%, at least 150%, or more lower than its reference value.

Likewise, in the context of the first method of the invention, the level of a biomarker is considered "increased" when the level of said biomarker in a sample is higher than a reference value. The levels of a biomarker are considered to be higher than its reference value when it is at least 1.5%, at least 2%, at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 100%, at least 110%, at least 120%, at least 130%, at least 140%, at least 150%, or more higher than its reference value.

In particular embodiment, the first method of the invention further comprises determining the presence or absence of apolipoprotein E type 4 isoform or a nucleic acid sequence encoding said apolipoprotein E type 4 isoform in a sample from the subject. In such a particular embodiment, the presence of apolipoprotein E type 4 isoform or a nucleic acid sequence encoding said apolipoprotein E type 4 isoform in said sample is indicative of a high likelihood of the subject developing Alzheimer's disease.

The term "apolipoprotein E" or "Apo E", as used herein, refers to a protein found in chylomicrons and intermediate-density lipoproteins (IDLs) that is essential for the normal catabolism of triglyceride-rich lipoprotein constituents. In humans, apolipoprotein E is encoded by the gene *ApoE,* which is a polymorphic gene with three major alleles, *ε2, ε3,* and *ε4*, which encodes the isoforms ApoE2 (cys112, cys158), ApoE3 (cys112, arg158), and ApoE4 (arg112, arg158). The apolipoprotein E can be from any origin, for example human, bovine, murine, equine, canine, etc., depending on the subject which is going to be diagnosed with the first method of the invention. In a particular embodiment, the Apo E is the human protein with the UniProt accession number P02649 (April 16, 2014).

The term "apolipoprotein E type 4 isoform" or "apolipoprotein E epsilon 4" or "apo E cod variable" or "apo E4", as used herein, refers to the isoform E4 of the protein apolipoprotein E, which is defined by the presence of the residue arginine in positions 112 and 158 of the amino acid sequence.

In a particular embodiment, the first method of the invention comprises determining the level of at least one biomarker selected from the biomarkers of table 1, preferably 21:0 ceramide, and determining the presence or absence of apolipoprotein E type 4 isoform or a nucleic acid sequence encoding said apolipoprotein E type 4 isoform.

In another particular embodiment, the first method of the invention comprises determining the level of at least two biomarkers selected from the biomarkers of table 1, preferably 21:0 ceramide and apolipoprotein C-1, and determining the presence or absence of apolipoprotein E type 4 isoform or a nucleic acid sequence encoding said apolipoprotein E type 4 isoform.

In another particular embodiment, the first method of the invention comprises determining the level of at least three biomarkers selected from the biomarkers of table 1, and determining the presence or absence of apolipoprotein E type 4 isoform or a nucleic acid sequence encoding said apolipoprotein E type 4 isoform. Preferably, the three biomarkers selected from the biomarkers of table 1 are one of the following combinations:
(i) 21:0 ceramide, apolipoprotein C-I and glutamic acid,
(ii) 21:0 ceramide, apolipoprotein C-I and alanine,
(iii)21:0 ceramide, apolipoprotein C-I and cortisol,
(iv)21:0 ceramide, apolipoprotein C-I and docosahexaenoic acid,
(v) 21:0 ceramide, apolipoprotein C-I and conjugated linoleic acid,
(vi)21:0 ceramide, apolipoprotein C-I and sarcosine,
(vii) 21:0 ceramide, apolipoprotein C-I and apolipoprotein C-II.

In another particular embodiment, the first method of the invention comprises determining the level of at least four biomarkers selected from the biomarkers of table 1, and determining the presence or absence of apolipoprotein E type 4 isoform or a nucleic acid sequence encoding said apolipoprotein E type 4 isoform. Preferably, the four biomarkers selected from the biomarkers of table 1 are one of the following combinations:
(i) 21:0 ceramide, apolipoprotein C-I, apolipoprotein C-II and cortisol,
(ii) 21:0 ceramide, apolipoprotein C-I, cortisol and glutamic acid,
(iii)21:0 ceramide, apolipoprotein C-I, cortisol and alanine.

In another particular embodiment, the first method of the invention comprises determining the level of at least five biomarkers selected from the biomarkers of table 1, preferably 21:0 ceramide, apolipoprotein C-I, cortisol, alanine and glutamic acid, and determining the presence or absence of apolipoprotein E type 4 isoform or a nucleic acid sequence encoding said apolipoprotein E type 4 isoform.

In another particular embodiment, the first method of the invention comprises determining the level of at least six biomarkers selected from the biomarkers of table 1, preferably 21:0 ceramide, apolipoprotein C-I, cortisol, alanine, glutamic acid and apolipoprotein CII, and determining the presence or absence of apolipoprotein E type 4 isoform or a nucleic acid sequence encoding said apolipoprotein E type 4 isoform.

In another particular embodiment, the first method of the invention comprises determining the level of at least seven biomarkers selected from the biomarkers of table 1, preferably 21:0 ceramide, apolipoprotein C-I, cortisol, alanine, glutamic acid, apolipoprotein CII and CLA, and determining the presence or absence of apolipoprotein E type 4 isoform or a nucleic acid sequence encoding said apolipoprotein E type 4 isoform.

In another particular embodiment, the first method of the invention comprises determining the level of at least eight biomarkers selected from the biomarkers of table 1, preferably 21:0 ceramide, apolipoprotein C-I, cortisol, alanine, glutamic acid, apolipoprotein CII, CLA and DHA, and determining the presence or absence of apolipoprotein E type 4 isoform or a nucleic acid sequence encoding said apolipoprotein E type 4 isoform.

In another particular embodiment, the first method of the invention comprises determining the level of all the biomarkers of table 1 and determining the presence or absence of apolipoprotein E type 4 isoform or a nucleic acid sequence encoding said apolipoprotein E type 4 isoform.

The presence or absence of apolipoprotein E type 4 isoform can be determined by any method known in the art suitable for the determination of the presence of the type 4 isoform of apolipoprotein E. in particular, the presence or absence of apolipoprotein E type 4 isoform can be determine by means of immunological method which comprises the use of an antibody which specifically recognizes the type 4 isoform of apolipoprotein E and does not recognizes the type 2 and 3 isoforms of apolipoprotein E, i.e., an antibody which binds to ApoE4 but exhibits essentially no binding to ApoE2 or ApoE3 in the same binding conditions.

Antibodies used to selectively or specifically ApoE4 can be produced by any suitable technique known by the skilled person. ApoE4 may be obtained from a human patient determined to be homozygous therefore, then purified and used as the immunogen for the production of monoclonal or polyclonal antibodies. Purified ApoE isoforms may be produced by recombinant means to express a biologically active isoform, or even an immunogenic fragment thereof may be used as an immunogen.

For this invention, an antibody selectively or specifically binding ApoE4 generally refers to a molecule capable of reacting with or otherwise recognizing or binding such a ligand. An antibody has binding affinity for a ligand or is specific for a ligand if the antibody binds or is capable of binding the ligand as measured or determined by standard antibody-antigen or ligand-receptor assays, for example, competitive assays, saturation assays, or standard immunoassays such as ELISA or RIA. This definition of specificity applies to single heavy and/or light chains, CDRs, fusion proteins or fragments of heavy and/or light chains, which are specific for the ligand if they bind the ligand alone or in combination.

Alternatively, the presence or absence of apolipoprotein E type 4 isoform can be detected by isoelectric focusing of the apolipoprotein E isolated from a sample of the patient. Isoelectric focusing is an electrophoretic technique by which the molecules are separated based on their isoelectric points (pI) along a continuous pH gradient. Reference proteins, commercially available, are used to indicate a gradient along which the sample proteins match up according to where their pH matches their pI. In Warnick, et al., Clin. Lab. Med., 2006, 26 (4):803-46, very-low-density apolipoproteins are isolated from plasma samples and applied to isoelectric focusing gels and the isoelectric focusing patterns of the ApoE isoforms are obtained. According to the Warnick et al. procedure, pI values of the ApoE isoforms, type 2, type 3 and type 4, were about 5.9, 6.0 and 6.1 respectively in 8M urea at 4° C.

The presence or absence of a nucleic acid encoding apolipoprotein E type 4 isoform can be determined in a sample from the patient by any method known in the art suitable for the determination of the presence of a nucleic acid encoding apolipoprotein E type 4 isoform. Any sample from the patient which contains nucleic acids from that subject may be employed, including tissue samples and blood samples. The amino acid sequences and nucleic acid sequences for ApoE4 are known and described. See, for example, Paik et al., Proc. Natl. Acad. Sci. U.S.A., 1985, 82:3445-3449 for the nucleic acid sequence of ApoE3.

Determining the presence or absence of DNA encoding an ApoE4 isoform may be carried out with an oligonucleotide probe labelled with a suitable detectable group, or by means of an amplification reaction such as a polymerase chain reaction or ligase chain reaction (the product of which amplification reaction may then be detected with a labelled oligonucleotide probe or a number of other techniques). Amplification of a selected, or target, nucleic acid sequence may be carried out by any suitable means. Polymerase chain reaction is currently preferred. DNA amplification techniques such as the foregoing can involve the use of a probe, a pair of probes, or two pairs of probes which specifically bind to DNA encoding ApoE4, but do not bind to DNA encoding ApoE2 or ApoE3 under the same hybridization conditions, and which serve as the primer or primers for the amplification of the ApoE4 DNA or a portion thereof in the amplification reaction. In general, an oligonucleotide probe which is used to detect DNA encoding ApoE4 is an oligonucleotide probe which binds to DNA encoding ApoE4, but does not bind to DNA encoding ApoE2 or ApoE3 under the same hybridization conditions.

According to this particular embodiment, the presence of apolipoprotein E type 4 isoform or a nucleic acid sequence encoding said apolipoprotein E type 4 isoform in said sample is indicative of a high likelihood of the subject developing Alzheimer's disease. The term "high likelihood" has been previously defined.

Additional particular embodiments of the first method of the invention include the determination of the expression level of a combination or panel of biomarkers, wherein said combination of biomarkers comprises:
(i) 21:0 ceramide, apolipoprotein C-I and glutamic acid,
(ii) 21:0 ceramide, apolipoprotein C-I and alanine,
(iii) 21:0 ceramide, apolipoprotein C-I and cortisol,
(iv) 21:0 ceramide, apolipoprotein C-I and docosahexaenoic acid,
(v) 21:0 ceramide, apolipoprotein C-I and conjugated linoleic acid,
(vi) 21:0 ceramide, apolipoprotein C-I and sarcosine,
(viii) 21:0 ceramide, apolipoprotein C-I and apolipoprotein C-II,
(ix) 21:0 ceramide, apolipoprotein C-I and apolipoprotein E type 4 isoform,
(x) 21:0 ceramide, apolipoprotein C-I, apolipoprotein C-II and cortisol,
(xi) 21:0 ceramide, apolipoprotein C-I, cortisol and glutamic acid,
(xii) 21:0 ceramide, apolipoprotein C-I, cortisol and alanine,
(xiii) 21:0 ceramide, apolipoprotein C-I, cortisol, glutamic acid and alanine,
(xiv) 21:0 ceramide, apolipoprotein C-I, cortisol, glutamic acid, alanine and apolipoprotein C-II,
(xv) 21:0 ceramide, apolipoprotein C-I, cortisol, glutamic acid, alanine, apolipoprotein C-II and conjugated linoleic acid,
(xvi) 21:0 ceramide, apolipoprotein C-I, cortisol, glutamic acid, alanine, apolipoprotein C-II, conjugated linoleic acid and docosahexaenoic acid and
(xvii) 21:0 ceramide, apolipoprotein C-I, cortisol, alanine, glutamic acid, apolipoprotein C-II, conjugated linoleic acid, docosahexaenoic acid and apolipoprotein E type 4 isoform.

The first method of the invention can be implemented in a computer system. Therefore, in a particular embodiment, the first method of the invention is implemented in a computer system.

In further aspects, the invention relates to a computer system that is provided with means for implementing the first method of the invention, to a computer program provided with means for implementing the first method of the invention and to a computer-readable data medium comprising said computer program.

The term "computer system", as used herein, refers to a system having a computer, where the computer comprises a computer-readable medium embodying software to operate the computer.

The first method of the invention can be implemented on a stand-alone computer or as part of a networked computer system. In a stand-alone computer, all the software and data can reside on local memory devices, for example an optical disk or flash memory device can be used to store the computer software for implementing the invention as well as the data. In alternative embodiments, the software or the data or both can be accessed through a network connection to remote devices. In one networked computer system embodiment, the invention use a client -server environment over a public network, such as the internet or a private network to connect to data and resources stored in remote and/or centrally located locations. In this embodiment, a server including a web server can provide access, either open access, pay as you go or subscription based access to the information provided according to the invention. In a client server environment, a client computer executing a client software or program, such as a web browser, connects to the server over a network. The client software or web browser provides a user interface for a user of the invention to input data and information and receive access to data and information. The client software can be viewed on a local computer display or other output device and can allow the user to input information, such as by using a computer keyboard, mouse or other input device. The server executes one or more computer programs that enable the client software to input data, process data according to the invention and output data to the user, as well as provide access to local and remote computer resources. For example, the user interface can include a graphical user interface comprising an access element, such as a text box, that permits entry of data from the assay, e.g., the levels of the biomarkers of a subject and/or the reference values for said biomarkers, as well as a display element that can provide a graphical read out of the results of a comparison with a score card, or data sets transmitted to or made available by a processor following execution of the instructions encoded on a computer-readable medium.

### Method for the diagnosis of Alzheimer's disease or mild cognitive impairment

In a second aspect, the invention relates to an *in vitro* method, hereinafter second method of the invention, for the diagnosis of Alzheimer's disease or mild cognitive impairment in a subject comprising
(a) determining in a sample from said subject the level of at least one biomarker selected from the biomarkers of Table 2, and
(b) comparing the level of said at least one biomarker with its reference value,
wherein decreased levels of at least one biomarker selected from the group consisting of 21:0 ceramide, apolipoprotein C-I, sarcosine, conjugated linoleic acid, docosahexaenoic acid and apolipoprotein C-II compared to its reference value and/or increased levels of the biomarker alanine compared to its reference value are indicative of the subject suffering from Alzheimer's disease or mild cognitive impairment.

The expression "method for the diagnosis of Alzheimer's disease or mild cognitive impairment", as used herein, refers to a method that consists essentially of the steps of the second method of the invention although it may include additional steps. "Diagnosing", as used herein, refers to evaluating the probability according to which a subject suffers from a disease, in particular from Alzheimer's disease or MCI. The method for the diagnosis of Alzheimer's disease or MCI of the invention is carried out *in vitro.*

The term "subject", as used herein, refers to all animals classified as mammals and includes, but is not restricted to, domestic and farm animals, primates and humans, e.g., human beings, non-human primates, cows, horses, pigs, sheep, goats, dogs, cats, or rodents. Preferably, the subject is a male or female human of any age or race.

The first step of the second method of the invention comprises the determination of de levels of at least one biomarker selected from the biomarkers of Table 2 in a sample from a subject.

**Table 2**

| **BIOMARKER** |
|---|
| 21:0 ceramide |
| Apolipoprotein C-I (Apo C-I) |
| Alanine |
| Sarcosine |
| Conjugated linoleic acid (CLA) |
| Docohexaenoic acid (DHA) |
| Apolipoprotein C-II (Apo C-II) |

The terms "Alzheimer's disease", "mild cognitive impairment" and "*in vitro*", "sample", "level", "biomarker", "21:0 ceramide", "apolipoprotein C-I", "sarcosine", "conjugated linoleic acid", "docosahexaenoic acid", "apolipoprotein C-II", "alanine", "decreased levels" and "increased levels" have been defined in connection with the first method of the invention. The preferred and particular embodiments of first method of the invention regarding these terms are also included in the second method of the invention.

The term "reference value", has been previously defined in connection with the first method of the invention. The reference value according to the second method of the invention can be obtained from one or more subjects who do not suffer from Alzheimer's disease or MCI (i.e., control subjects).

The second method of the invention comprises determining at least one biomarker selected from the biomarkers of Table 2. In a particular embodiment, said at least one biomarker is 21:0 ceramide. In another particular embodiment, said at least one biomarker is apolipoprotein C-I. In another particular embodiment, said at least one biomarker is alanine. In another particular embodiment, said at least one biomarker is sarcosine. In another particular embodiment, said at least one biomarker is CLA. In another particular embodiment, said at least one biomarker is DHA. In another particular embodiment, said at least one biomarker is apolipoprotein C-II.

In a particular embodiment, the second method of the invention comprises determining at least two biomarkers selected from the biomarkers of Table 2. In a preferred embodiment, said two biomarkers are 21:0 ceramide and a biomarker selected from apolipoprotein C-I, alanine, sarcosine, CLA, DHA and apolipoprotein C-II. In a more preferred embodiment, said two biomarkers are 21:0 ceramide and apolipoprotein C-II.

In another particular embodiment, the second method of the invention comprises determining at least three biomarkers selected from the biomarkers of Table 2. In a preferred embodiment, said three biomarkers are 21:0 ceramide, apolipoprotein C-I and one biomarker selected from alanine, sarcosine, CLA, DHA and apolipoprotein C-II. In a particular embodiment, said three biomarkers are:
(i) 21:0 ceramide, apolipoprotein C-I and alanine,
(ii) 21:0 ceramide, apolipoprotein C-I and docosahexaenoic acid,
(iii)21:0 ceramide, apolipoprotein C-I and conjugated linoleic acid,
(iv)21:0 ceramide, apolipoprotein C-I and sarcosine,
(v) 21:0 ceramide, apolipoprotein C-I and apolipoprotein C-II.

In another particular embodiment, the second method of the invention comprises determining at least four biomarkers selected from the biomarkers of Table 2. In a preferred embodiment, said four biomarkers are 21:0ceramide, apolipoprotein C-I and two biomarkers selected from alanine, sarcosine, CLA, DHA, apolipoprotein C-II.

In another particular embodiment, the second method of the invention comprises determining at least five biomarkers selected from the biomarkers of Table 2. In a preferred embodiment, said five biomarkers are 21:0 ceramide, apolipoprotein C-I and three biomarkers selected from alanine, sarcosine, CLA, DHA and apolipoprotein C-II.

In another particular embodiment, the second method of the invention comprises determining at least six biomarkers selected from the biomarkers of Table 2. In a preferred embodiment, said six biomarkers are 21:0 ceramide, apolipoprotein C-I and four biomarkers selected from alanine, sarcosine, CLA, DHA and apolipoprotein C-II.

In another particular embodiment, the second method of the invention comprises determining all the biomarkers of Table 2.

In a particular embodiment, when the second method of the invention comprises determining at least one, at least two, at least three, at least four, at least five or at least six biomarkers, any additional biomarker is not selected from the biomarkers of Table 2.

In a particular embodiment, the second method of the invention further comprises
(a) determining in a sample from said subject the levels of at least one additional biomarker selected from glutamic acid and cortisol and
(b) comparing the level of said additional biomarker with its reference value, wherein decreased levels of glutamic acid compared to its reference value and/or increased levels of cortisol compared with its reference value are indicative of the subject suffering from Alzheimer's disease or mild cognitive impairment.

In a particular embodiment, the additional biomarker is glutamic acid. In another particular embodiment, the additional biomarker is cortisol.

In a particular embodiment of the second method of the invention, the following combinations of biomarkers are determined:
(i) 21:0 ceramide, apolipoprotein C-I and glutamic acid,
(ii) 21:0 ceramide, apolipoprotein C-I and alanine,
(iii) 21:0 ceramide, apolipoprotein C-I and cortisol,
(iv) 21:0 ceramide, apolipoprotein C-I and docosahexaenoic acid,
(v) 21:0 ceramide, apolipoprotein C-I and conjugated linoleic acid,
(vi) 21:0 ceramide, apolipoprotein C-I and sarcosine,
(vii) 21:0 ceramide, apolipoprotein C-I and apolipoprotein C-II,
(viii) 21:0 ceramide, apolipoprotein C-I, apolipoprotein C-II and cortisol,
(ix) 21:0 ceramide, apolipoprotein C-I, cortisol and glutamic acid,
(x) 21:0 ceramide, apolipoprotein C-I, cortisol and alanine,
(xi) 21:0 ceramide, apolipoprotein C-I, cortisol, glutamic acid and alanine,
(xii) 21:0 ceramide, apolipoprotein C-I, cortisol, glutamic acid, alanine and apolipoprotein C-II,
(xiii) 21:0 ceramide, apolipoprotein C-I, cortisol, glutamic acid, alanine, apolipoprotein C-II and conjugated linoleic acid,
(xiv) 21:0 ceramide, apolipoprotein C-I, cortisol, glutamic acid, alanine, apolipoprotein C-II, conjugated linoleic acid and docosahexaenoic acid.

The level of the biomarkers according to the second method of the invention can be determined by the same methods previously explained for determining the level of the biomarkers according to the first method of the invention.

In a particular embodiment, the second method of the invention further comprises determining the presence or absence of apolipoprotein E type 4 isoform or a nucleic acid sequence encoding said apolipoprotein E type 4 isoform in a sample from the subject. In such a particular embodiment, the presence of apolipoprotein E type 4 isoform or a nucleic acid sequence encoding said apolipoprotein E type 4 isoform in said sample is indicative of the subject suffering from Alzheimer's disease or MCI. The term "apolipoprotein E type 4 isoform" as well as methods for determining the presence or absence of apolipoprotein E type 4 isoform or a nucleic acid encoding apolipoprotein E type 4 isoform have been previously defined in connection with the first method of the invention.

Additional particular embodiments of the second method of the invention include the determination of the following combination of biomarkers:
(i) 21:0 ceramide, apolipoprotein C-I and apolipoprotein E type 4 isoform,
(ii) 21:0 ceramide, apolipoprotein C-I, cortisol, alanine, glutamic acid, apolipoprotein C-II, conjugated linoleic acid, docosahexaenoic acid and apolipoprotein E type 4 isoform.

The second method of the invention can be implemented in a computer system. Therefore, in a particular embodiment, the second method of the invention is implemented in a computer system.

In further aspects, the invention relates to a computer system that is provided with means for implementing the second method of the invention, to a computer program provided with means for implementing the second method of the invention and to a computer-readable data medium comprising said computer program.

The terms "computer system", "computer program" and "computer-readable data" have been defined in connection with the first method of the invention.

### Method for the treatment and/or prevention of Alzheimer's disease

According to the first aspect of the invention, the determination of a series of markers allows the identification of patients with MCI and who have a high probability of developing Alzheimer's disease. Therefore, this information can be used for the identification of patients which would benefit from the treatment with a therapy aimed at preventing the appearance of Alzheimer's disease. Thus, in another aspect, the invention relates to a method for the treatment and/or prevention of Alzheimer's disease, hereinafter third method of the invention, comprising administering a therapy for the treatment of Alzheimer's disease to a patient with mild cognitive impairment, wherein the patient is selected for said treatment if a sample from said patient contains decreased levels of at least one biomarker selected from the group consisting of 21:0 ceramide, apolipoprotein C-I, sarcosine, conjugated linoleic acid, docosahexaenoic acid, apolipoprotein C-II and glutamic acid compared to its reference value and/or contains increased levels of at least one biomarker selected from the group consisting of alanine and cortisol compared to its reference value.

The terms "Alzheimer's disease", "patient", "mild cognitive impairment", "sample", "decreased levels", "increased levels", "biomarker", "21:0 ceramide", "apolipoprotein C-I", "sarcosine", "conjugated linoleic acid", "docosahexaenoic acid", "apolipoprotein C-II", "alanine" and "reference value" have been defined in connection with the first method of the invention. The preferred and particular embodiments of first method of the invention regarding these terms are also included in the third method of the invention.

The expression "treatment and/or prevention", as used herein, refers to both therapeutic measures and prophylactic or preventive measures, wherein the object is to prevent or slow down (lessen) an undesired physiological change or disorder, such as Alzheimer's disease. Beneficial or desired clinical results include, but are not limited to, alleviation of symptoms, diminishment of extent of disease, stabilized (i.e., not worsening) state of disease, delay or slowing of disease progression, amelioration or palliation of the disease state, and remission (whether partial or total), whether detectable or undetectable. "Treatment and/or prevention" can also mean prolonging survival as compared to expected survival if not receiving treatment. Those in need of treatment include those already with the condition or disorder as well as those prone to have the condition or disorder or those in which the condition or disorder is to be prevented.

The term "therapy for the treatment of Alzheimer's disease", as used herein, refers to any therapy directed to alleviate the symptoms, diminish the extent of the disease, stabilize (i.e., not worsen) the state of disease, delay or slow disease progression, ameliorate or palliate the disease state, or achieve remission (whether partial or total) of the disease.

There are two main types of therapies used to treat Alzheimer's disease: cholinesterase inhibitors and NMDA receptor antagonists. Cholinesterase inhibitors include donepezil hydrochloride (Aricept), rivastigmine (Exelon) and galantamine (Reminyl) and are directed to prevent the breakdown of acetylcholine by acetylcholinesterase, improving the function of brain cells. The NMDA receptor antagonist memantine (Ebixa) bind to NMDA receptors on brain cells blocking the activity of glutamate, which is released in increased amounts in brain cells of patients with Alzheimer's disease.

Other therapies described for the treatment and/or prevention of Alzheimer's disease are directed to reduce the toxic effects caused by the deposition of the amyloid beta peptide (β-amyloid, AB or Aβ) that comes from amyloid precursor protein (APP). For example, WO09087568A describes compositions including compounds such as kercetin, bipigenine and kaemfenol that reduce neuronal death caused by exposure to Aβ peptide. US5948800A describes the use of drugs to prevent or treat AD, these drugs contain the active compound 2-phenyl-1, 2-benzisoselenazol-3 (2H)-one, whose effect is based on the reduction of neuronal toxicity caused by the peptide Aβ. US2002102259 describes a method for inhibiting Aβ peptide effects in the brain of an animal comprising administering a compound that effectively modulates the activity of CD45.

In a particular embodiment, the therapy for the treatment of Alzheimer's disease that is administered according to the third method of the invention is a cholinesterase inhibitor. In another particular embodiment, the therapy for the treatment of Alzheimer's disease that is administered according to the third method of the invention is a NMDA receptor antagonist.

According to the third method of the invention, a therapy for the treatment of Alzheimer's disease is administered to a patient with MCI, being the patient selected for said treatment if a sample from said patient contains significant different levels of at least one biomarker selected from the biomarkers of Table 1 compared to its reference value. According to the third method of the invention, said significant different levels can be decreased levels of at least one biomarker selected from the group consisting of 21:0 ceramide, apolipoprotein C-I, sarcosine, conjugated linoleic acid, docosahexaenoic acid, apolipoprotein C-II and glutamic acid compared to its reference value and/or increased levels of at least one biomarker selected from the group consisting of alanine and cortisol compared to its reference value. In a particular embodiment, said at least one biomarker is 21:0 ceramide. In another particular embodiment, said at least one biomarker is apolipoprotein C-I. In another particular embodiment, said at least one biomarker is alanine. In another particular embodiment, said at least one biomarker is sarcosine. In another particular embodiment, said at least one biomarker is CLA. In another particular embodiment, said at least one biomarker is DHA. In another particular embodiment, said at least one biomarker is apolipoprotein C-II. In another particular embodiment, said at least one biomarker is glutamic acid. In another particular embodiment, said at least one biomarker is cortisol.

In a particular embodiment, the patient is selected for the therapy if a sample from said patient contains significant different levels of at least two biomarkers selected from the biomarkers of Table 1 compared to their reference value. In a preferred embodiment, said two biomarkers are 21:0 ceramide and a biomarker selected from apolipoprotein C-I, alanine, sarcosine, CLA, DHA, apolipoprotein C-II, glutamic acid and cortisol. In a more preferred embodiment, said two biomarkers are 21:0 ceramide and apolipoprotein C-II.

In another particular embodiment, the patient is selected for the therapy if a sample from said patient contains significant different levels of at least three biomarkers selected from the biomarkers of Table 1 compared to their reference value. In a preferred embodiment, said three biomarkers are 21:0 ceramide, apolipoprotein C-I and one biomarker selected from alanine, sarcosine, CLA, DHA, apolipoprotein C-II, glutamic acid and cortisol. In a particular embodiment, said three biomarkers are:
(i) 21:0 ceramide, apolipoprotein C-I and glutamic acid,
(ii) 21:0 ceramide, apolipoprotein C-I and alanine,
(iii)21:0 ceramide, apolipoprotein C-I and cortisol,
(iv)21:0 ceramide, apolipoprotein C-I and docosahexaenoic acid,
(v) 21:0 ceramide, apolipoprotein C-I and conjugated linoleic acid,
(vi)21:0 ceramide, apolipoprotein C-I and sarcosine,
(vii) 21:0 ceramide, apolipoprotein C-I and apolipoprotein C-II.

In another particular embodiment, the patient is selected for the therapy if a sample from said patient contains significant different levels of at least four biomarkers selected from the biomarkers of Table 1 compared to their reference value. In a preferred embodiment, said four biomarkers are 21:0ceramide, apolipoprotein C-I and two biomarkers selected from alanine, sarcosine, CLA, DHA, apolipoprotein C-II, glutamic acid and cortisol. In a particular embodiment, said four biomarkers are 21:0ceramide, apolipoprotein C-I, cortisol and one biomarker selected from alanine, sarcosine, CLA, DHA, apolipoprotein C-II and glutamic acid. In a more particular embodiment, said four biomarkers are:
(i) 21:0 ceramide, apolipoprotein C-I, apolipoprotein C-II and cortisol,
(ii) 21:0 ceramide, apolipoprotein C-I, cortisol and glutamic acid,
(iii)21:0 ceramide, apolipoprotein C-I, cortisol and alanine.

In another particular embodiment, the patient is selected for the therapy if a sample from said patient contains significant different levels of at least five biomarkers selected from the biomarkers of Table 1 compared to their reference value. In a preferred embodiment, said five biomarkers are 21:0 ceramide, apolipoprotein C-I and three biomarkers selected from alanine, sarcosine, CLA, DHA, apolipoprotein C-II, glutamic acid and cortisol. In a particular embodiment, said five biomarkers are 21:0 ceramide, apolipoprotein C-I, cortisol and two biomarkers selected from alanine, sarcosine, CLA, DHA, apolipoprotein C-II and glutamic acid. In a more particular embodiment, said five biomarkers are 21:0 ceramide, apolipoprotein C-I, cortisol, alanine and one biomarker selected from sarcosine, CLA, DHA, apolipoprotein C-II and glutamic acid. In another more particular embodiment, said five biomarkers are 21:0 ceramide, apolipoprotein C-I, cortisol, glutamic acid and one biomarker selected from alanine, sarcosine, CLA, DHA and apolipoprotein C-II. In an even more particular embodiment, said five biomarkers are 21:0 ceramide, apolipoprotein C-I, cortisol, alanine and glutamic acid.

In another particular embodiment, the patient is selected for the therapy if a sample from said patient contains significant different levels of at least six biomarkers selected from the biomarkers of Table 1 compared to their reference value. In a preferred embodiment, said six biomarkers are 21:0 ceramide, apolipoprotein C-I and four biomarkers selected from alanine, sarcosine, CLA, DHA, apolipoprotein C-II, glutamic acid and cortisol. In a particular embodiment, said six biomarkers are 21:0 ceramide, apolipoprotein C-I, cortisol and three biomarkers selected from alanine, sarcosine, CLA, DHA, apolipoprotein C-II and glutamic acid. In a more particular embodiment, said six biomarkers are 21:0 ceramide, apolipoprotein C-I, cortisol, alanine and two biomarkers selected from sarcosine, CLA, DHA, apolipoprotein C-II and glutamic acid. In a more particular embodiment, said six biomarkers are 21:0 ceramide, apolipoprotein C-I, cortisol, alanine, glutamic acid and one biomarker selected from sarcosine, CLA, DHA and apolipoprotein C-II. In an even more particular embodiment, said six biomarkers are 21:0 ceramide, apolipoprotein C-I, cortisol, alanine, glutamic acid and apolipoprotein C-II.

In another particular embodiment, the patient is selected for the therapy if a sample from said patient contains significant different levels of at least seven biomarkers selected from the biomarkers of Table 1 compared to their reference value. In a preferred embodiment, said seven biomarkers are 21:0 ceramide, apolipoprotein C-I and five biomarkers selected from alanine, sarcosine, CLA, DHA, apolipoprotein C-II, glutamic acid and cortisol. In a particular embodiment, said seven biomarkers are 21:0 ceramide, apolipoprotein C-I, cortisol and four biomarkers selected from alanine, sarcosine, CLA, DHA, apolipoprotein C-II and glutamic acid. In a more particular embodiment, said seven biomarkers are 21:0 ceramide, apolipoprotein C-I, cortisol, alanine and three biomarkers selected from sarcosine, CLA, DHA, apolipoprotein C-II and glutamic acid. In a more particular embodiment, said seven biomarkers are 21:0 ceramide, apolipoprotein C-I, cortisol, alanine, glutamic acid and two biomarkers selected from sarcosine, CLA, DHA and apolipoprotein C-II. In a more particular embodiment, said seven biomarkers are 21:0 ceramide, apolipoprotein C-I, cortisol, alanine, glutamic acid, apolipoprotein C-II and one biomarker selected from sarcosine, CLA and DHA. In an even more particular embodiment, said seven biomarkers are 21:0 ceramide, apolipoprotein C-I, cortisol, alanine, glutamic acid, apolipoprotein C-II and CLA.

In another particular embodiment, the patient is selected for the therapy if a sample from said patient contains significant different levels of at least eight biomarkers selected from the biomarkers of Table 1 compared to their reference value. In a preferred embodiment, said eight biomarkers are 21:0 ceramide, apolipoprotein C-I and six biomarkers selected from alanine, sarcosine, CLA, DHA, apolipoprotein C-II, glutamic acid and cortisol. In a particular embodiment, said eight biomarkers are 21:0 ceramide, apolipoprotein C-I, cortisol and five biomarkers selected from alanine, sarcosine, CLA, DHA, apolipoprotein C-II and glutamic acid. In a more particular embodiment, said eight biomarkers are 21:0 ceramide, apolipoprotein C-I, cortisol, alanine and four biomarkers selected from sarcosine, CLA, DHA, apolipoprotein C-II and glutamic acid. In a more particular embodiment, said eight biomarkers are 21:0 ceramide, apolipoprotein C-I, cortisol, alanine, glutamic acid and three biomarkers selected from sarcosine, CLA, DHA and apolipoprotein C-II. In a more particular embodiment, said eight biomarkers are 21:0 ceramide, apolipoprotein C-I, cortisol, alanine, glutamic acid, apolipoprotein C-II and two biomarkers selected from sarcosine, CLA and DHA. In a more particular embodiment, said eight biomarkers are 21:0 ceramide, apolipoprotein C-I, cortisol, alanine, glutamic acid, apolipoprotein C-II, CLA and one biomarker selected from sarcosine and DHA. In an even more particular embodiment, said eight biomarkers are 21:0 ceramide, apolipoprotein C-I, cortisol, alanine, glutamic acid, apolipoprotein C-II, CLA and DHA.

In another particular embodiment, the patient is selected for the therapy if a sample from said patient contains significant different levels all the biomarkers of Table 1 compared to their reference value.

In particular embodiment, the sample of the patient selected for the therapy further comprises determining the apolipoprotein E type 4 isoform or a nucleic acid sequence encoding said apolipoprotein E type 4 isoform.

The term "apolipoprotein E type 4 isoform" has been previously defined.

In a particular embodiment, the patient is selected for the therapy if a sample from said patient contains significant different levels of at least one biomarker selected from the biomarkers of Table 1 compared to its reference value, preferably 21:0 ceramide, and further comprises apolipoprotein E type 4 isoform or a nucleic acid sequence encoding said apolipoprotein E type 4 isoform.

In a particular embodiment, the patient is selected for the therapy if a sample from said patient contains significant different levels of at least two biomarkers selected from the biomarkers of Table 1 compared to their reference value, preferably 21:0 ceramide and apolipoprotein C-1, and further comprises apolipoprotein E type 4 isoform or a nucleic acid sequence encoding said apolipoprotein E type 4 isoform.

In a particular embodiment, the patient is selected for the therapy if a sample from said patient contains significant different levels of at least three biomarkers selected from the biomarkers of Table 1 compared to their reference value, and further comprises apolipoprotein E type 4 isoform or a nucleic acid sequence encoding said apolipoprotein E type 4 isoform. Preferably, the three biomarkers selected from the biomarkers of table 1 are one of the following combinations:
(i) 21:0 ceramide, apolipoprotein C-I and glutamic acid,
(ii) 21:0 ceramide, apolipoprotein C-I and alanine,
(iii) 21:0 ceramide, apolipoprotein C-I and cortisol,
(iv) 21:0 ceramide, apolipoprotein C-I and docosahexaenoic acid,
(v) 21:0 ceramide, apolipoprotein C-I and conjugated linoleic acid,
(vi) 21:0 ceramide, apolipoprotein C-I and sarcosine,
(vii)21:0 ceramide, apolipoprotein C-I and apolipoprotein C-II.

In a particular embodiment, the patient is selected for the therapy if a sample from said patient contains significant different levels of at least four biomarkers selected from the biomarkers of Table 1 compared to their reference value, and further comprises apolipoprotein E type 4 isoform or a nucleic acid sequence encoding said apolipoprotein E type 4 isoform. Preferably, the four biomarkers selected from the biomarkers of table 1 are one of the following combinations:
(i) 21:0 ceramide, apolipoprotein C-I, apolipoprotein C-II and cortisol,
(ii) 21:0 ceramide, apolipoprotein C-I, cortisol and glutamic acid,
(iii)21:0 ceramide, apolipoprotein C-I, cortisol and alanine.

In a particular embodiment, the patient is selected for the therapy if a sample from said patient contains significant different levels of at least five biomarkers selected from the biomarkers of Table 1 compared to their reference value, preferably 21:0 ceramide, apolipoprotein C-I, cortisol, alanine and glutamic acid, and further comprises apolipoprotein E type 4 isoform or a nucleic acid sequence encoding said apolipoprotein E type 4 isoform.

In a particular embodiment, the patient is selected for the therapy if a sample from said patient contains significant different levels of at least six biomarkers selected from the biomarkers of Table 1 compared to their reference value, preferably 21:0 ceramide, apolipoprotein C-I, cortisol, alanine, glutamic acid and apolipoprotein CII, and further comprises apolipoprotein E type 4 isoform or a nucleic acid sequence encoding said apolipoprotein E type 4 isoform.

In a particular embodiment, the patient is selected for the therapy if a sample from said patient contains significant different levels of at least seven biomarkers selected from the biomarkers of Table 1 compared to their reference value, preferably 21:0 ceramide, apolipoprotein C-I, cortisol, alanine, glutamic acid, apolipoprotein CII and CLA, and further comprises apolipoprotein E type 4 isoform or a nucleic acid sequence encoding said apolipoprotein E type 4 isoform.

In a particular embodiment, the patient is selected for the therapy if a sample from said patient contains significant different levels of at least eight biomarkers selected from the biomarkers of Table 1 compared to their reference value, 21:0 ceramide, apolipoprotein C-I, cortisol, alanine, glutamic acid, apolipoprotein CII, CLA and DHA, and further comprises apolipoprotein E type 4 isoform or a nucleic acid sequence encoding said apolipoprotein E type 4 isoform.

In a particular embodiment, the patient is selected for the therapy if a sample from said patient contains significant different levels of all the biomarkers of Table 1 compared to their reference value, 21:0 ceramide, apolipoprotein C-I, cortisol, alanine, glutamic acid, apolipoprotein CII, CLA and DHA, and further comprises apolipoprotein E type 4 isoform or a nucleic acid sequence encoding said apolipoprotein E type 4 isoform.

Further aspects of the invention are as follows:
1. An *in vitro* method for determining the likelihood that a patient with mild cognitive impairment develops Alzheimer's disease comprising
   (a) determining in a sample from said patient the level of at least one biomarker selected from the biomarkers of Table 1, and
   (b) comparing the level of said at least one biomarker with its reference value, wherein decreased levels of at least one biomarker selected from the group consisting of 21:0 ceramide, apolipoprotein C-I, sarcosine, conjugated linoleic acid, docosahexaenoic acid, apolipoprotein C-II and glutamic acid compared to its reference value and/or increased levels of at least one biomarker selected from the group consisting of alanine and cortisol compared to its reference value are indicative of a high likelihood of the subject developing Alzheimer's disease.
2. The method according to aspect 1, wherein the at least one biomarker is 21:0 ceramide.
3. The method according to aspects 1 or 2 comprising determining at least two biomarkers selected from the biomarkers of Table 1.
4. The method according to aspect 3 wherein the at least two biomarkers are 21:0 ceramide and apolipoprotein C-I.
5. The method according to aspect 4 wherein the following combinations of markers are determined:
   (i) 21:0 ceramide, apolipoprotein C-I and glutamic acid,
   (ii) 21:0 ceramide, apolipoprotein C-I and alanine,
   (iii) 21:0 ceramide, apolipoprotein C-I and cortisol,
   (iv) 21:0 ceramide, apolipoprotein C-I and docosahexaenoic acid,
   (v) 21:0 ceramide, apolipoprotein C-I and conjugated linoleic acid,
   (vi) 21:0 ceramide, apolipoprotein C-I and sarcosine,
   (vii) 21:0 ceramide, apolipoprotein C-I and apolipoprotein C-II,
   (viii) 21:0 ceramide, apolipoprotein C-I, apolipoprotein C-II and cortisol,
   (ix) 21:0 ceramide, apolipoprotein C-I, cortisol and glutamic acid,
   (x) 21:0 ceramide, apolipoprotein C-I, cortisol and alanine,
   (xi) 21:0 ceramide, apolipoprotein C-I, cortisol, glutamic acid and alanine,
   (xii) 21:0 ceramide, apolipoprotein C-I, cortisol, glutamic acid, alanine and apolipoprotein C-II,
   (xiii) 21:0 ceramide, apolipoprotein C-I, cortisol, glutamic acid, alanine, apolipoprotein C-II and conjugated linoleic acid,
   (xiv) 21:0 ceramide, apolipoprotein C-I, cortisol, glutamic acid, alanine, apolipoprotein C-II, conjugated linoleic acid and docosahexaenoic acid.
6. The method according to any of aspects 1 to 5 further comprising determining the presence or absence of apolipoprotein E type 4 isoform or a nucleic acid sequence encoding said apolipoprotein E type 4 isoform in a sample from said subject, wherein the presence of apolipoprotein E type 4 isoform or a nucleic acid sequence encoding said apolipoprotein E type 4 isoform in said sample is indicative of a high likelihood of the subject developing Alzheimer's disease.
7. An *in vitro* method for the diagnosis of Alzheimer's disease or mild cognitive impairment in a subject comprising
   (a) determining in a sample from said subject the level of at least one biomarker selected from the biomarkers of Table 2, and
   (b) comparing the level of said at least one biomarker with its reference value, wherein decreased levels of at least one biomarker selected from the group consisting of 21:0 ceramide, apolipoprotein C-I, sarcosine, conjugated linoleic acid, docosahexaenoic acid and apolipoprotein C-II compared to its reference value and/or increased levels of the biomarker alanine compared to its reference value are indicative of the subject suffering from Alzheimer's disease or mild cognitive impairment.
8. The method according to aspect 7 further comprising
   (a) determining in a sample from said subject the levels of at least one additional biomarker selected from glutamic acid and cortisol and
   (b) comparing the level of said additional biomarker with its reference value, wherein decreased levels of glutamic acid compared to its reference value and/or increased levels of cortisol compared with its reference value are indicative of the subject suffering from Alzheimer's disease or mild cognitive impairment.
9. The method according to aspects 7 or 8 wherein the at least one biomarker is 21:0 ceramide.
10. The method according to any of aspects 7 to 9 comprising determining at least two biomarkers selected from the biomarkers of Table 2.
11. The method according to aspects 10 wherein the at least two biomarkers are 21:0 ceramide and apolipoprotein C-I.
12. The method according to aspect 11 wherein the following combinations of markers are determined:
   (i) 21:0 ceramide, apolipoprotein C-I and glutamic acid,
   (ii) 21:0 ceramide, apolipoprotein C-I and alanine,
   (iii) 21:0 ceramide, apolipoprotein C-I and cortisol,
   (iv) 21:0 ceramide, apolipoprotein C-I and docosahexaenoic acid,
   (v) 21:0 ceramide, apolipoprotein C-I and conjugated linoleic acid,
   (vi) 21:0 ceramide, apolipoprotein C-I and sarcosine,
   (vii) 21:0 ceramide, apolipoprotein C-I and apolipoprotein C-II,
   (viii) 21:0 ceramide, apolipoprotein C-I, apolipoprotein C-II and cortisol,
   (ix) 21:0 ceramide, apolipoprotein C-I, cortisol and glutamic acid,
   (x) 21:0 ceramide, apolipoprotein C-I, cortisol and alanine,
   (xi) 21:0 ceramide, apolipoprotein C-I, cortisol, glutamic acid and alanine,
   (xii) 21:0 ceramide, apolipoprotein C-I, cortisol, glutamic acid, alanine and apolipoprotein C-II,
   (xiii) 21:0 ceramide, apolipoprotein C-I, cortisol, glutamic acid, alanine, apolipoprotein C-II and conjugated linoleic acid,
   (xiv) 21:0 ceramide, apolipoprotein C-I, cortisol, glutamic acid, alanine, apolipoprotein C-II, conjugated linoleic acid and docosahexaenoic acid.
13. The method according to any of aspect 7 to 12 further comprising determining the presence or absence of apolipoprotein E type 4 isoform or a nucleic acid sequence encoding said apolipoprotein E type 4 isoform in a sample from said subject, wherein the presence of apolipoprotein E type 4 isoform or a nucleic acid sequence encoding said apolipoprotein E type 4 isoform in said sample is indicative of the subject suffering from Alzheimer's disease or mild cognitive impairment.
14. The method according to any of aspects 1 to 13 wherein the mild cognitive impairment is an amnestic mild cognitive impairment.
15. The method according to any of aspects 1 to 14 wherein the Alzheimer's disease is prodromal Alzheimer disease.
16. The method according to any of aspects 1 to 15 wherein the sample is blood, serum or plasma.
17. The method according to any of aspects 1 to 16 implemented in a computer system or a computer system or computer program provided with means for implementing the methods according to any of claims 1 to 16, or a computer-readable data medium comprising said computer program.
18. A method for the treatment and/or prevention of Alzheimer's disease comprising administering a therapy for the treatment of Alzheimer's disease to a patient with mild cognitive impairment, wherein the patient is selected for said treatment if a sample from said patient contains decreased levels of at least one biomarker selected from the group consisting of 21:0 ceramide, apolipoprotein C-I, sarcosine, conjugated linoleic acid, docosahexaenoic acid, apolipoprotein C-II and glutamic acid compared to its reference value and/or contains increased levels of at least one biomarker selected from the group consisting of alanine and cortisol compared to its reference value.
19. The method according to aspect 18 wherein the at least one biomarker is 21:0 ceramide.
20. The method according to aspect 18 or 19 wherein at least two biomarkers selected from the biomarkers of Table 1.
21. The method according to aspect 20 wherein the at least two biomarkers are 21:0 ceramide and apolipoprotein C-I.
22. The method according to aspect 21 wherein the biomarkers are:
   (i) 21:0 ceramide, apolipoprotein C-I and glutamic acid,
   (ii) 21:0 ceramide, apolipoprotein C-I and alanine,
   (iii) 21:0 ceramide, apolipoprotein C-I and cortisol,
   (iv) 21:0 ceramide, apolipoprotein C-I and docosahexaenoic acid,
   (v) 21:0 ceramide, apolipoprotein C-I and conjugated linoleic acid,
   (vi) 21:0 ceramide, apolipoprotein C-I and sarcosine,
   (vii) 21:0 ceramide, apolipoprotein C-I and apolipoprotein C-II,
   (viii) 21:0 ceramide, apolipoprotein C-I, apolipoprotein C-II and cortisol,
   (ix) 21:0 ceramide, apolipoprotein C-I, cortisol and glutamic acid,
   (x) 21:0 ceramide, apolipoprotein C-I, cortisol and alanine,
   (xi) 21:0 ceramide, apolipoprotein C-I, cortisol, glutamic acid and alanine,
   (xii) 21:0 ceramide, apolipoprotein C-I, cortisol, glutamic acid, alanine and apolipoprotein C-II,
   (xiii) 21:0 ceramide, apolipoprotein C-I, cortisol, glutamic acid, alanine, apolipoprotein C-II and conjugated linoleic acid, and
   (xiv) 21:0 ceramide, apolipoprotein C-I, cortisol, glutamic acid, alanine, apolipoprotein C-II, conjugated linoleic acid and docosahexaenoic acid.
23. The method according to any of aspect 18 to 22 wherein the sample of the patient further contains the apolipoprotein E type 4 isoform or a nucleic acid sequence encoding said apolipoprotein E type 4 isoform.
24. The method according to any of aspect 18 to 23 wherein the sample is blood, serum or plasma.

### EXAMPLES

### Metabolite analysis

### 1. UPLC/MS metabolite analysis

### Material and methods

In metabolic profiling, there is no single platform or method to analyze the entire metabolome of a biological sample mainly due to the wide concentration range of metabolites coupled to their extensive chemical diversity. Therefore, metabolite extraction was accomplished by fractionating the plasma samples into pools of species with similar physicochemical properties, using appropriate combinations of organic solvents. Previously, plasma was extracted from blood samples by using EDTA tubes and 5 minutes and 1700 x g centrifugation. Supernatants were collected and centrifuged at 13000 rpm for 10 min. Supernatants were collected and frozen before analysis Three methods were used according to the target analytes' chemical class:
- *Platform 1: Fatty acyls, bile acids, and lysoglycerophospholipids profiling.* Proteins were precipitated from the defrosted plasma samples (75 µL) by adding 4 volumes of methanol in 1.5 mL microtubes at room temperature. The methanol used for extraction was spiked with the following compounds not detected in unspiked human serum extracts using the same method: tryptophan-d5(indole-d5), PC(13:0/0:0), NEFA(19:0), and dihydrocholic acid. After brief vortex mixing the samples were incubated overnight at -20°C. Supernatants (300 µL) were collected after centrifugation at 16,000 x g for 15 minutes, dried and reconstituted in 75 µL methanol before being transferred to vials for UPLC®-MS-TOF analysis
- *Platform* 2: *Amino acids profiling.* 10 µl aliquots from the extracts prepared for *Platform 1* were transferred to microtubes and derivatised for amino acid analysis before UPLC®-MS-SQD analysis.
- *Platform 3: Glycerolipids, cholesterol esters, sphingolipids and glycerophospholipids profiling.* 10 µL plasma extracts were mixed with 10 µL sodium chloride (50 mM) and 110 µL of chloroform / methanol (2:1) in 1.5 mL microtubes at room temperature. The extraction solvent was spiked with the following compounds not detected in unspiked human serum extracts by platform 3: SM(d18:1/6:0), PE(17:0/17:0), PC(19:0/19:0), TAG(13:0/13:0/13:0), TAG(17:0/17:0/17:0), Cer(d18:1/17:0), ChoE(12:0). After brief vortex mixing, the samples were incubated for 1 hour at -20 °C. After centrifugation at 16,000 x g for 15 minutes, 70 mL of the lower organic phase was collected and the solvent removed. The dried extracts were then reconstituted in 100 µL acetronitrile/ isopropanol (50:50), centrifuged (16,000 x g for 5 minutes), and transferred to vials for UPLC®-MS-TOF analysis.

A different UPLC®-MS method was used for each platform; chromatographic separation and mass spectrometric detection conditions employed are summarized in Table 3.

**Table 3. UPLC-MS analysis methods**

| | **Platform 1** | **Platform 2** | **Platform 3** |
|---|---|---|---|
| **Column type** | UPLC BEH C18, 1.0 x 100 mm, 1.7 mm | UPLC BEH C18, 1.0 x 100 mm, 1.7 mm | UPLC BEH C18, 2.1 x 100 mm, 1.7 mm |
| **Flow rate** | 0.14 ml/minute | 0.14 ml/minute | 0.40 ml/minute |
| **Solvent A** | H₂O + 0.05 % Formic acid | 10 mM Ammonium Bicarbonate (pH 8.8) | H₂O + ACN + 10 mM Ammonium Formate |
| **Solvent B** | ACN + 0.05 % Formic acid | ACN | ACN + isopropanol + Formato amónico 10 mM |
| **(%B), Time** | 0%, 0 minutes | 2%, 0 minutes | 40%, 0 minutes |
| **(%B), Time** | 50%, 2 minutes | 8%, 6.5 minutes | 100%, 10 minutes |
| **(%B), Time** | 100%, 13 minutes | 20%, 10 minutes | 40%, 15 minutes |
| **(%B), Time** | 0%, 18 minutes | 30%, 11 minutes | 40%, 17 minutes |
| **(%B), Time** | - | 100%, 12 minutes | - |
| **(%B), Time** | - | 2%, 14 minutes | - |
| **Column temperature** | 40 °C | 40 °C | 60 °C |
| **Injection volume** | 2 ml | 2 ml | 3 ml |
| **Source temperature** | 120°C | 120°C | 120°C |
| **Nebulisation N₂ flow** | 600 l/hour | 600 l/hour | 1000 l/hour |
| **Nebulisation N₂ temperature** | 350 °C | 350 °C | 500 °C |
| **Cone N₂ flow** | 30 l/hour | 10 l/hour | 30 l/hour |
| **Capilary voltage** | 2,8 kV | 3,2 kV | 3,2 kV |
| **Cone voltage** | 50V | 30V | 30V |

### Quality control

A test mixture of standard compounds (Acetaminophen, Sulfaguanidine, Sulfadimethoxine, Val-Tyr-Val, Terfenadine, Leucine-Enkephaline, Reserpine and Erythromicyn - all 5 nM in water) was analyzed before and after the entire set of randomized, duplicated sample injections in order to examine the retention time stability (generally < 6 s variation, injection-to-injection), mass accuracy [platforms 1 and 3 (generally < 3 ppm for *m*/*z* 400-1000, and < 1.2 mDa for *m*/*z* 50-400)] and sensitivity of the system throughout the course of the run which lasted a maximum of 34 h per batch of samples injected. For each injection batch, the overall quality of the analysis procedure was monitored using five repeat extracts of the QC Validation sample.

Additionally, two different types of quality control (QC) samples were used to assess the data quality. The QC samples are reference plasma samples, which are evenly distributed over the batches and extracted and analyzed at the same time as the individual samples.
- QC Calibration sample: used to correct the different response factors between and within batches.
- QC Validation sample: used to assess how well data pre-processing procedure improved the data quality.

For each of the three analytical platforms, randomized duplicate sample injections were performed, with each of the QC calibration and validation extracts uniformly interspersed throughout the entire batch run.

All data were processed using the TargetLynx application manager for MassLynx 4.1 software (Waters Corp., Milford, USA). A set of predefined retention time, mass-to-charge ratio pairs, Rt-m/z, corresponding to metabolites included in the analysis are fed into the program. Associated extracted ion chromatograms (mass tolerance window = 0.05 Da) are then peak-detected and noise-reduced in both the LC and MS domains such that only true metabolite related features are processed by the software. A list of chromatographic peak areas is then generated for each sample injection.

For identified metabolites, representative MS detection response curves were generated using an internal standard for each chemical class included in the analysis. By assuming similar detector response levels for all metabolites belonging to a given chemical class, this allowed a linear detection range to be defined for each variable. Maximum values were defined as those at which the detector response became nonlinear with respect to the concentration of the representative internal standard. Variables were not considered for further analysis where more than 30% of data points were found outside their corresponding linear detection range. For example, variables that used to be excluded from the serum o plasma analysis at this stage were the most abundant species detected in that kind of bio fluids, e.g. ChoE(18:2) [blood concentration ~2 mM], PC(16:0/0:0) [blood concentrations ~ 0.1 mM].

Normalization factors were calculated for each metabolite by dividing their intensities in each sample by the recorded intensity of an appropriate internal standard in that same sample:
The most appropriate internal standard for each variable was defined as that which resulted in a minimum relative standard deviation after correction, as calculated from the QC calibration samples over all the analysis batches. In general, as one would have expected, best internal standard trends followed chemical structural similarities between spiked compounds and endogenous variables.
   - Linear regression (internal standard corrected response as a function of sample injection order) was used to estimate in the QC calibration samples any intra-batch drift not corrected for by internal standard correction. For all variables, internal standard corrected response in each batch was divided by its corresponding intra-batch drift trend, such that normalized abundance values of the study samples were expressed with respect to the batch averaged QC calibration serum samples (arbitrarily set to 1).

Following normalization, the concordance between duplicate sample injection response values was assessed. Where coefficients of variation > 30% were found, corresponding sample injection data were returned for manual inspection of the automated integration performed by the TargetLynx software, and modifications performed where appropriate. Any remaining sample injection variable response zero values in the corrected dataset were replaced with missing values before averaging to form the final dataset that was used for study sample statistical analyses.

### 2. APOC-I ELISA analysis

### Summary

Apolipoprotein C-I (ApoC-I) is a 6.6 kDa apolipoprotein that is expressed primarily in the liver and activated when monocytes differentiate into macrophages. The *AssayMax Human Apolipoprotein C-I ELISA* kit is used for detection of human ApoC-I in plasma and serum samples. This assay employs a polyclonal antibody specific for human ApoC-I pre-coated onto a 96-well microplate with removablestrips. ApoC-I in standards and samples are sandwiched by the immobilized antibody and biotinylated polyclonal antibody specific for ApoC-I.

### Materials and methods

Serum samples were collected into a serum separator tube (EDTA tubes) and centrifuged at 3000 x g for 10 minutes. Samples were diluted 1:100 into EIA Diluent. It is necessary avoid repeated freeze-thaw cycles.

All reagents, working standards and samples were prepared as instructed. All reagents were brought to room temperature before use. The assay was performed at room temperature (20-30°C). 50 µl of Human ApoC-I standard or sample were added per well and then wells were covered with a sealing tape and incubated for 2 hours. Timer was started after the last sample addition. After wash five times with 200 µl of Wash Buffer manually, the plate was inverted each time and decanted the contents and hit 4-5 times on absorbent material to completely remove the liquid. 50 µl of Biotinylated Human ApoC-I antibody was added to each well and incubated for 2 hours before to wash the microplate as described above. 50 µl of Streptavidin-Peroxidase conjugate was added to each well and incubated for 30 minutes. After wash the microplate as described previously, 50 µl of Chromogen Substrate (tetramethylbenzidine) was added per well and incubated for about 30 minutes or till the optimal blue color density developed. Plate was tapped gently to ensure thorough mixing and break the bubbles in the well with pipette tip. Finally, 50 µl of Stop Solution (0.5 N hydrochloric acid) were added to each well and the absorbance was read on a microplate reader at a wavelength of 450 nm immediately.

### 3. APOC-II and APOC-III immunoturbidimetric analysis

### Summary

Quantitative determination of human apolipoprotein CII and human apoliprotein CIII, components both of VLDL and chylomicrons, were realised by immunoturbidimetric assay through the use of *K-ASSAY*® Apo CII and *K-ASSAY*® Apo CIII test kits, respectively.

### Material and methods

According to kits user guides, 4 ml serum sample was mixed with 300 µL Tris(hydroxymethyl)aminomethane (Reagent 1) and then incubated at 37°C for 5 minutes. When sample was mixed with 100 µL anti-human apolipoprotein CII antiserum or anti-human apolipoprotein CIII antiserum (Reagent 2) for 5 minutes at 37°C, agglutination was caused by the antigen-antibody reaction. The turbidity was measured at 450 nm with Hitachi 717 and Apo CII and Apo CIII in the sample were quantitatively determined. Human serum resuspended in 1 mL deionized water was used as control. For calibration, 5 different calibrators (B-F) were prepared using dissolved serum by adding saline solution (table 4). All reagents should be stored refrigerated (2-8°C).

**Table 4. Calibration serum dilutions**

| | **B** | **C** | **D** | **E** | **F** |
|---|---|---|---|---|---|
| **Dilution** | 1/10 | 2/10 | 4/10 | 6/10 | 1 |
| **Dissolved serum (mL)** | 50 | 100 | 200 | 300 | 350 |
| **Diluent (mL)** | 450 | 400 | 300 | 200 | 0 |
| **Total (mL)** | 500 | 500 | 500 | 500 | 500 |

### 4. Cortisol chemiluminiscent analysis

### Summary

IMMULITE® and IMMULITE® 1000 analyzers were used for quantitative measurement of cortisol by solid-phase, competitive chemiluminescent enzyme inmunoassay. Cortisol (hydrocortisone) is the most abundant circulating steroid and the major glucocorticoid secreted by the adrenal cortex. Physiologically effective in antiinflammatory activity and blood pressure maintenance, cortisol is also involved in gluconeogenesis, calcium absorption and the secretion of acid gastric and pepsin.

### Materials and methods

Preparation, setup, dilutions, adjustment, assay and quality control procedures were realized according to INMULITE® and INMULITE® 1000 operator's manual. For each sample, 10 mL serum and the following reagents were required:
- **Cortisol Test Units (LCO1):** Bead coated with polyclonal rabbit anticortisol antibody.
- **Cortisol Reagent Wedge (LCO2):** 7.5 ml alkaline phosphatase (bovine calf intestine) conjugated to cortisol in buffer, with preservative.
- **Cortisol Adjustors (LCOL, LCOH):** Two vials of 3 mL each, of cortisol in processed human serum, with preservative.
- **Cortisol Sample diluent (LCOZ):** For the dilution of patient samples, one vial of 25 mL of cortisol-free human serum, with preservative.
- **LSUBX:** Chemiluminescent substrate.
- **LPWS2:** Probe wash module.
- **LKPM:** Probe cleaning kit.
- **LCHx-y:** Sample cup holders (barcoded)
- **LSCP:** Sample cups (disposable).
- **LSCC:** Sample cup caps (optional).
- **CON6:** Tri-level multi-constituent control.
- Pipettes, deionized or distilled water and controls.

### 5. Data statistical analysis

The study consisted of 304 individuals who were classified as either being healthy (31%) or having Alzheimer's (33%), aMCI (19%) A further 17 % of individuals were classified as other dementias or undetermined. Information was analyzed from 517 variables.

The primary aim of the study was to determine classification rules for the following classification groups based upon the class variable:
1. Alzheimer vs. Healthy Control
2. aMCI vs. Healthy Control

Univariate analysis was initially undertaken for each comparison group to investigate the variables within each parameter group that had a significant association at the 5% level with an individual's classification. For continuous variables, a t-test was implemented whilst for categorical variables a chi-squared test was undertaken.

Focusing on variables found to be significant at the 5% level from the univariate analysis, a logistic regression model was built for each of the parameter groups. Stepwise selection was utilised to determine final models using an entry criteria equalling p<0.1 and stay criteria equalling p<0.05. As these models contain variables from only one parameter group, they are referred to throughout the analysis as single models. The final variables from each model were then included within a logistic regression model and stepwise selection was then used to determine the final models (referred to throughout the analysis as combined models).

The performance of these models was evaluated further by using a more conservative method. Cross-validation was utilised for each classification group as a robust method to determine the variables that are significantly associated with an individual's classification, independent of the sample of data being analysed. The cross-validations consisted of 100 random samples using a 70% sample for the training dataset, whilst the remaining 30% sample of data was used for validation. For each of the cross-validations, a logistic regression assuming stepwise selection was used to determine the final model with the frequency of times a variable is included within the final model being recorded.

Those variables found within at least 25% of final models were then included within a logistic regression model using stepwise selection to determine the final model. To enable determination of accuracy rates for each classification group based upon these classification rules, a further cross-validation was undertaken for logistic regression models including the final variables found within the classification rules. The cross-validations again consisted of 100 random samples using a 70% sample for the training dataset, whilst the remaining 30% sample of data was used for validation. Doing so, for each comparison group, the average and 95% confidence intervals of these accuracy rates was determined.

### Abbreviations

*ACN:* Acetonitrile
*ApoC-I:* Apolipoprotein C-I
*ApoC-II:* Apolipoprotein C-II
*ApoC-III:* Apolipoprotein C-III
*AUC:* Area Under Curve
*Cer:* Ceramide
*ChoE:* Cholesterol ester
*EDTA:* Ethylenediaminetetraacetic acid
*ELISA:* Enzyme-Linked ImmunoSorbent Assay
*HDL:* High Density Lipoprotein
*IDL:* Intermediate Density Lipoprotein
*LDL:* Low Density Lipoprotein
*MS:* Mass Spectrometry
*NEFA:* Non-Esterified Fatty Acid
*PE:* Phosphatidylethanolamine
*PC:* Phosphatidylcholine
*ROC:* Receiver Operating Characteristic
*SM:* Sphingomyelin
*SQD:* Single Quadrupole
*TAG:* Triacylglycerol
*TOF:* Time Of Flight
*Tyr:* Tyrosine
*UPLC:* Ultra Performance Liquid Chromatography
*Val:* Valine
*VLDL:* Very Low Density Lipoprotein

### Results

### 1. Summary

An in vitro method for the diagnosis of amnestic MCI (aMCI) and Alzheimer's disease and determining the likelihood of a patient with mild cognitive impairment of developing Alzheimer's disease has been defined following the analysis of 517 variables from 251 individuals divided in 3 groups previously classified by clinical guidelines (aMCI Criteria described by Petersen et. al. Arch Neurol. 1999 Mar;56(3):303-8; and Alzheimer's disease criteria by *NINCDS-ADRDA describes by* McKhann et al. Neurology. 1984 Jul;34(7):939-44): 93 individuals classified as Healthy Control, 100 individuals classified as Alzheimer's and 58 individuals classified as aMCI. Due to undetermined values obtained in cortisol analysis, 5 individuals from Healthy Control group and 1 individual from Alzheimer's disease group were excluded from this variable.

### 2. Key results

According to statics criteria, a limited number of variables comprised into at least one of final models were obtained:
- Ceramide(d18:1/21:0) (Cer(d18:1/21:0))
- Apolipoprotein C-I (APO C-I)
- Glutamic acid
- Docosahexaenoic acid (DHA)
- Alanine
- Conjugated linoleic acid (CLA)
- Sarcosine
- Apolipoprotein C-II (APO C-II)
- APOE e4 carriers (APOE cod)
- Cortisol

Cer(d:18:1/21:0), APO C-I, Glutamic acid, DHA, CLA, Sarcosine, APO C-II levels decreased into aMCI group respect Healthy Controls (table 5). Whereas, Alanine and Cortisol levels increased into aMCI patients respect Healthy Controls (table 7). The same tendency was observed when Alzheimer group is compared to Healthy Controls (table 9). Apolipoprotein E type 4 isoform or a nucleic acid sequence encoding said apolipoprotein E type 4 isoform (APO E cod) results were summarized in contingency tables (tables 6 and 8). Positive results are obtained when APO E cod is present and absence is noted by negative results. As tables 6 and 8 shown, the proportion of positive e4 carriers into Healthy Control was significantly lower than Alzheimer and aMCI respectively.

| MARKER | COMPARISON GROUP | n | Mean | SD | p value |
|---|---|---|---|---|---|
| Cer(d18:1/21:0) | Healthy Control | 93 | 1.959 | 0.958 | 0.003 |
| | Alzheimer | 100 | 1.601 | 0.681 | |
| APO C-I | Healthy Control | 93 | 0.056 | 0.018 | <0.001 |
| | Alzheimer | 100 | 0.044 | 0.025 | |
| Glutamic acid | Healthy Control | 93 | 0.114 | 0.046 | <0.001 |
| | Alzheimer | 100 | 0.073 | 0.029 | |
| DHA | Healthy Control | 93 | 1.741 | 0.805 | <0.001 |
| | Alzheimer | 100 | 1.349 | 0.683 | |
| Alanine | Healthy Control | 93 | 0.502 | 0.133 | <0.001 |
| | Alzheimer | 100 | 0.579 | 0.173 | |
| CLA | Healthy Control | 93 | 0.310 | 0.169 | 0.003 |
| | Alzheimer | 100 | 0.247 | 0.116 | |
| Sarcosine | Healthy Control | 93 | 0.966 | 0.148 | <0.001 |
| | Alzheimer | 100 | 0.851 | 0.167 | |
| APO C-II | Healthy Control | 93 | 0.487 | 0.229 | <0.001 |
| | Alzheimer | 100 | 0.322 | 0.211 | |
| Cortisol | Healthy Control | 88 | 13.542 | 4.444 | <0.001 |
| | Alzheimer | 99 | 21.866 | 6.829 | |
| Table 5. Mean and SD values of selected variables into Healthy Control and Alzheimer groups and corresponding p values obtained for this comparison analysis | | | | | |

| MARKER | COMPARISON GROUP | YES | NO | p value |
|---|---|---|---|---|
| APO E cod | Healthy Control | 26 | 98 | <0.001 |
| | Alzheimer | 60 | 40 | |
| Table 6. Contingency table of APO e4 carriers for Healthy Control vs Alzheimer comparison. | | | | |

| MARKER | COMPARISON GROUP | n | Mean | SD | p value |
|---|---|---|---|---|---|
| Cer(d18:1/21:0) | Healthy Control | 93 | 1.959 | 0.958 | <0.001 |
| | aMCI | 58 | 1.512 | 0.599 | |
| APO C-I | Healthy Control | 93 | 0.056 | 0.018 | 0.005 |
| | aMCI | 58 | 0.044 | 0.028 | |
| Glutamic acid | Healthy Control | 93 | 0.114 | 0.046 | 0.001 |
| | aMCI | 58 | 0.089 | 0.047 | |
| DHA | Healthy Control | 93 | 1.741 | 0.805 | 0.004 |
| | aMCI | 58 | 1.367 | 0.730 | |
| Alanine | Healthy Control | 93 | 0.502 | 0.133 | 0.008 |
| | aMCI | 58 | 0.589 | 0.219 | |
| CLA | Healthy Control | 93 | 0.310 | 0.169 | 0.075 |
| | aMCI | 58 | 0.262 | 0.151 | |
| Sarcosine | Healthy Control | 93 | 0.966 | 0.148 | 0.839 |
| | aMCI | 58 | 0.960 | 0.169 | |
| APO C-II | Healthy Control | 93 | 0.487 | 0.229 | <0.001 |
| | aMCI | 58 | 0.339 | 0.182 | |
| Cortisol | Healthy Control | 88 | 13.542 | 4.444 | <0.001 |
| | aMCI | 58 | 20.113 | 8.192 | |
| Table 7. Mean and SD values of selected variables into Healthy Control and aMCI groups and corresponding p values obtained for this comparison analysis. | | | | | |

| MARKER | COMPARISON GROUP | YES | NO | p value |
|---|---|---|---|---|
| APO E cod | Healthy Control | 26 | 98 | <0.001 |
| | aMCI | 25 | 33 | |
| Table 8. Contingency table of APO e4 carriers for Healthy Control vs aMCI comparison. | | | | |

The performance of a model to discriminate Healthy Controls from aMCI or Alzheimer was evaluated using receiver operating characteristic (ROC) curves. Each ROC curve represents a series of points with a sensitivity/specificity pairing based on plotting true positive rate (sensitivity) as a function of the false positive rate (specificity) at a particular threshold setting. The ability to distinguish between two groups is measured by the area under the ROC curve (AUC).

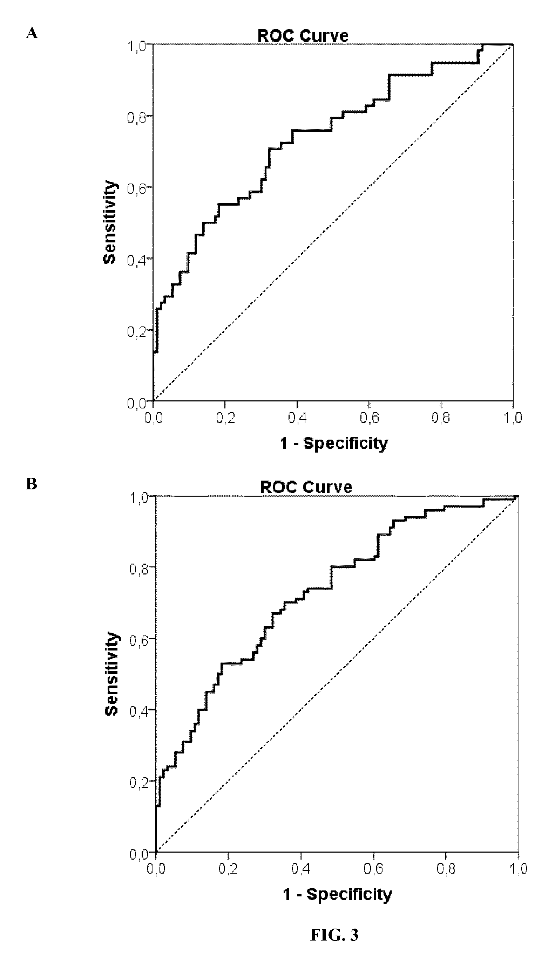

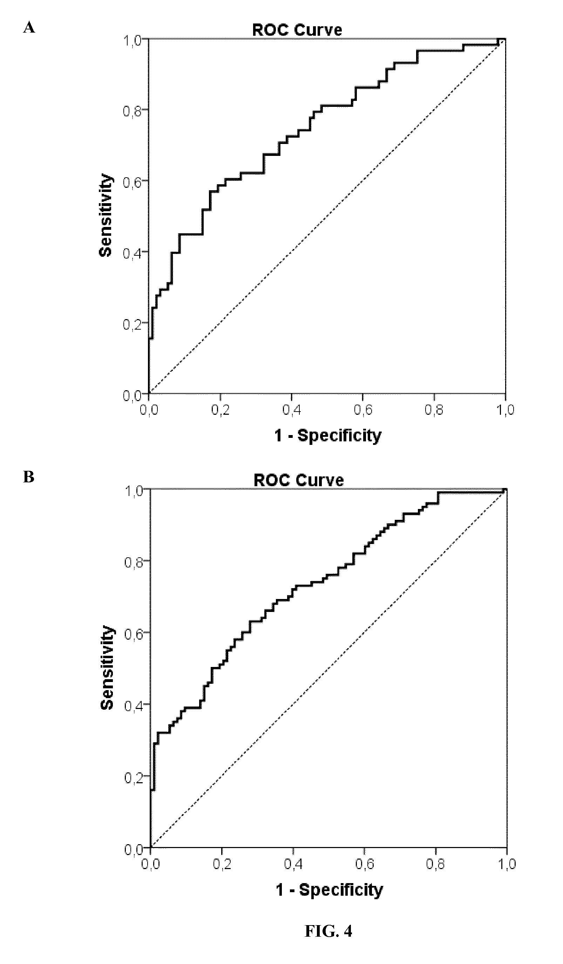

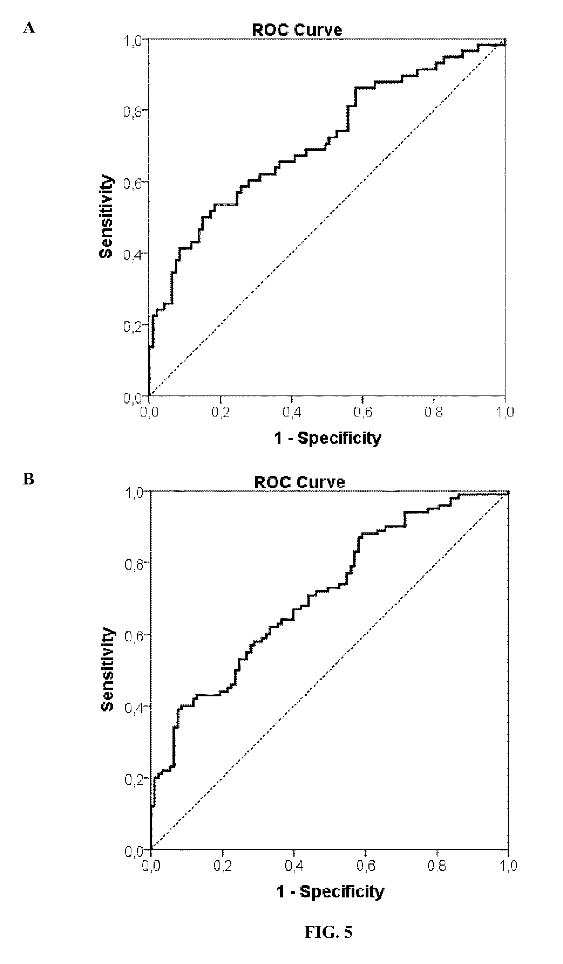

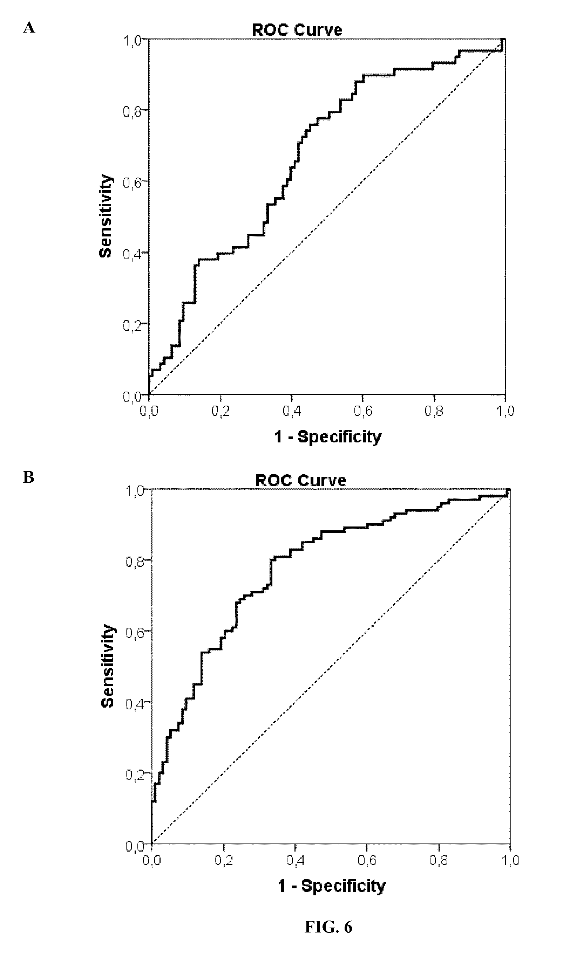

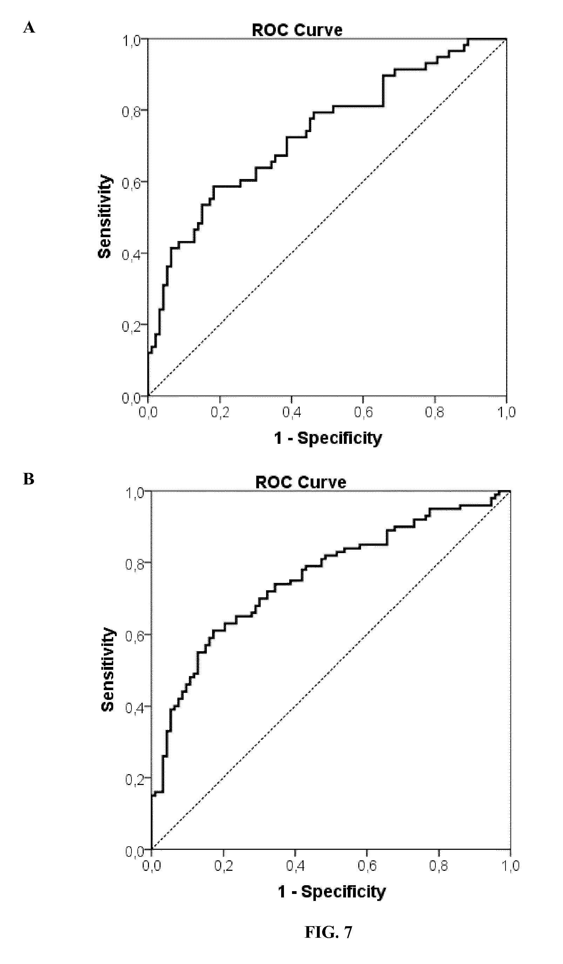

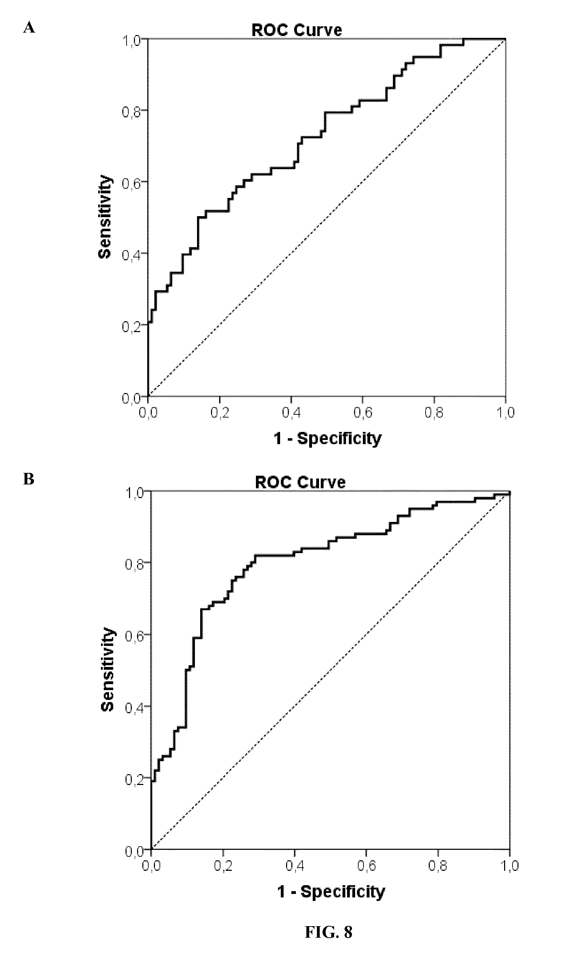

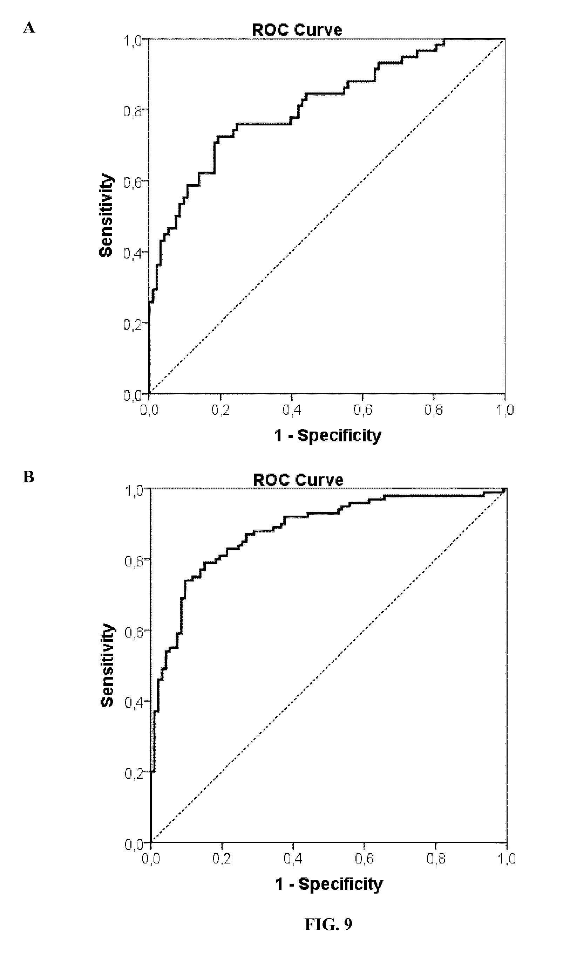

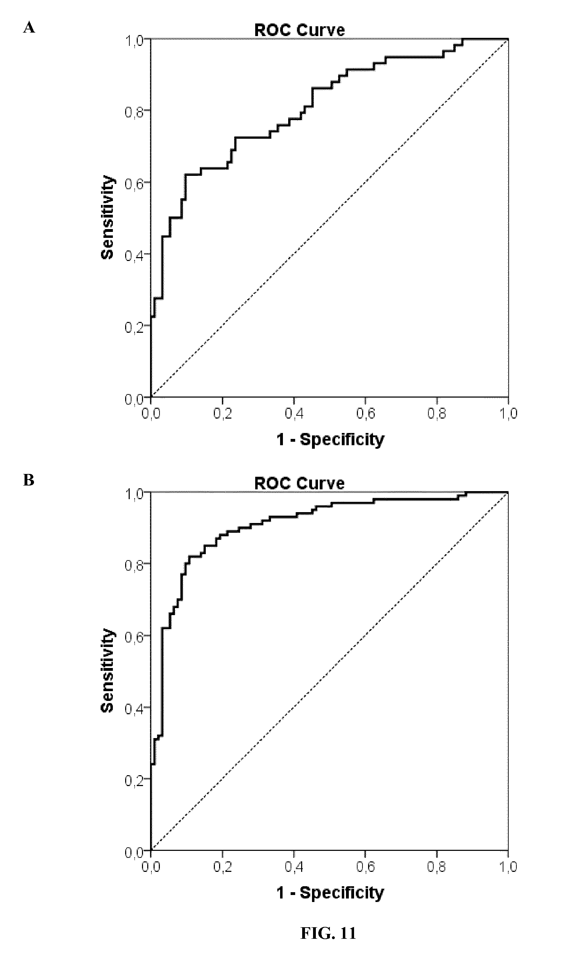

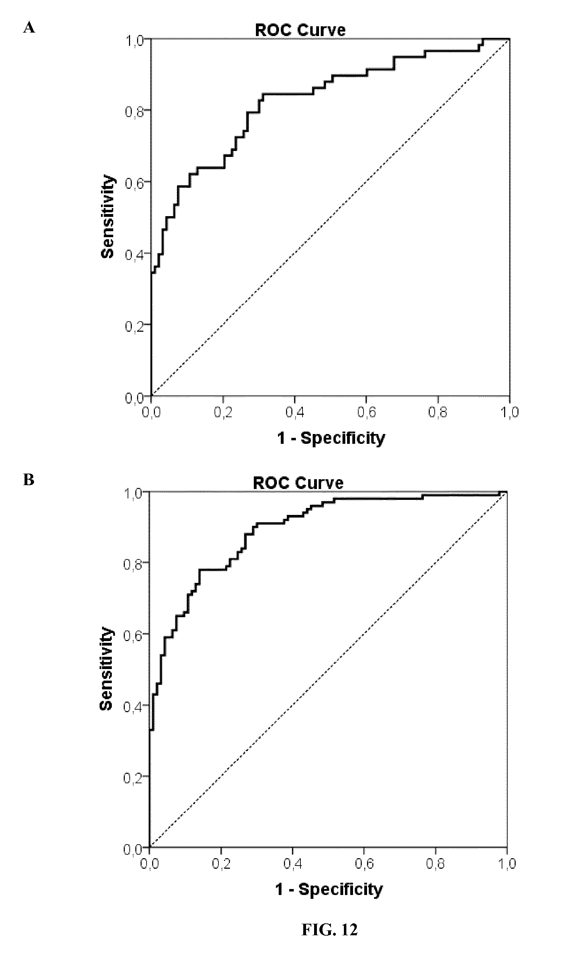

A series of classification rules comprise of different number of variables found within these final models. The corresponding ROC curves are provided (figures 1-17). Corresponding p values and AUC values are summarized in table 9.

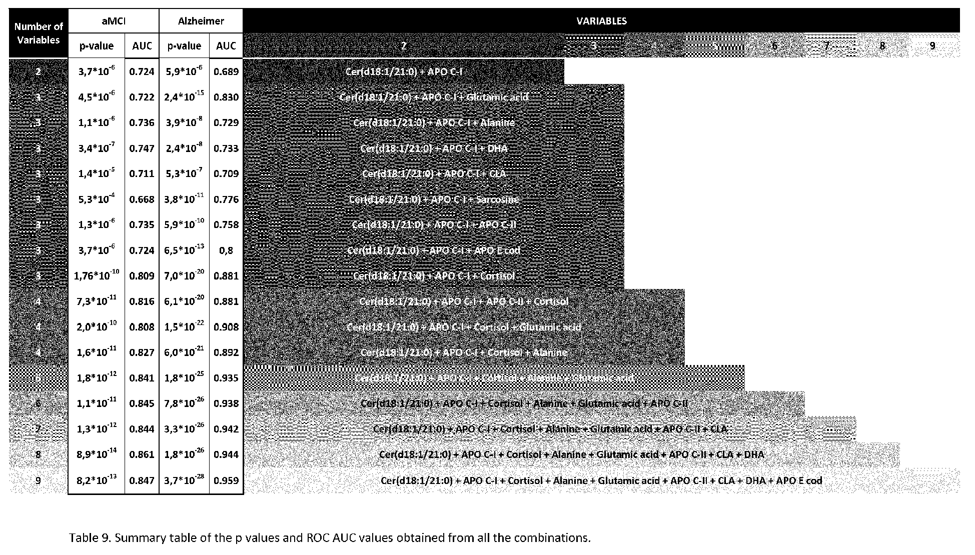

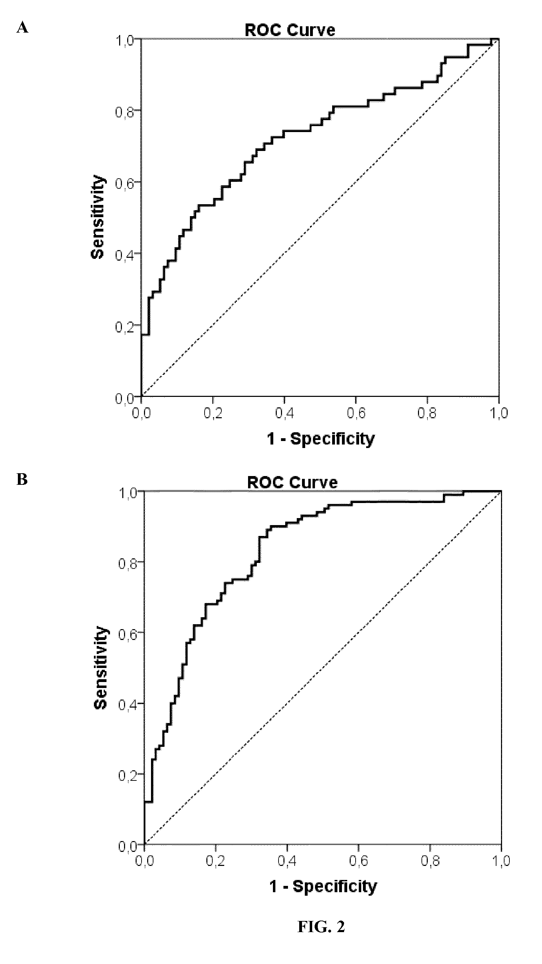

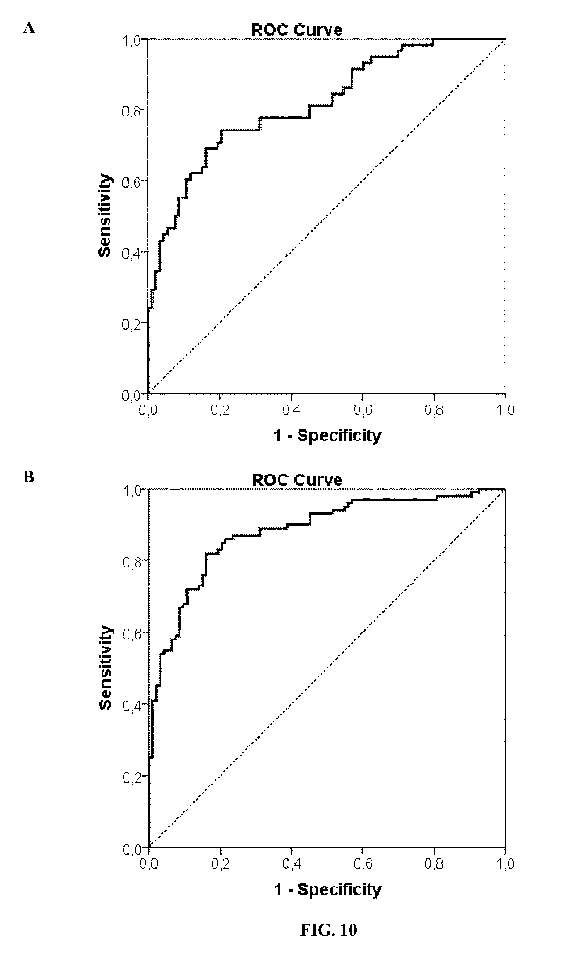

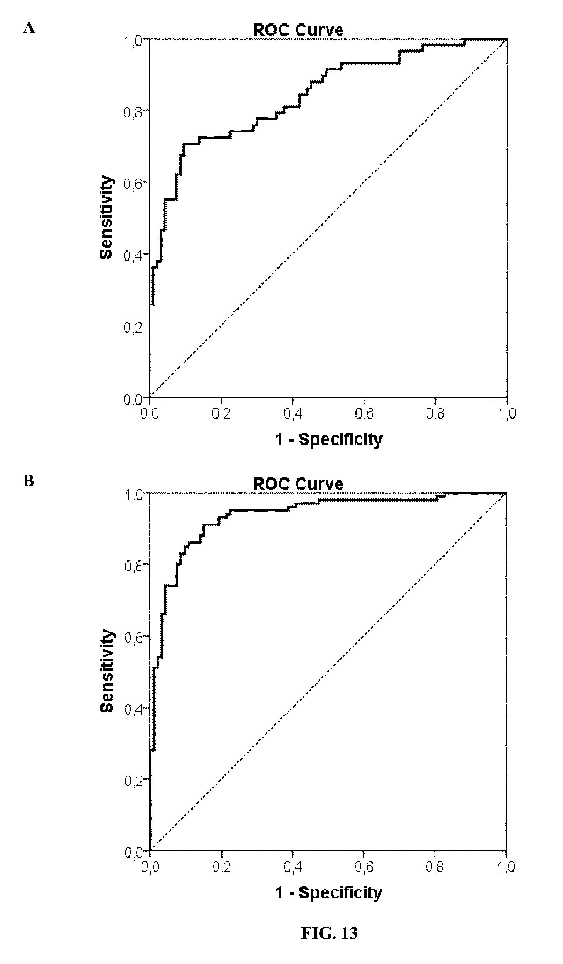

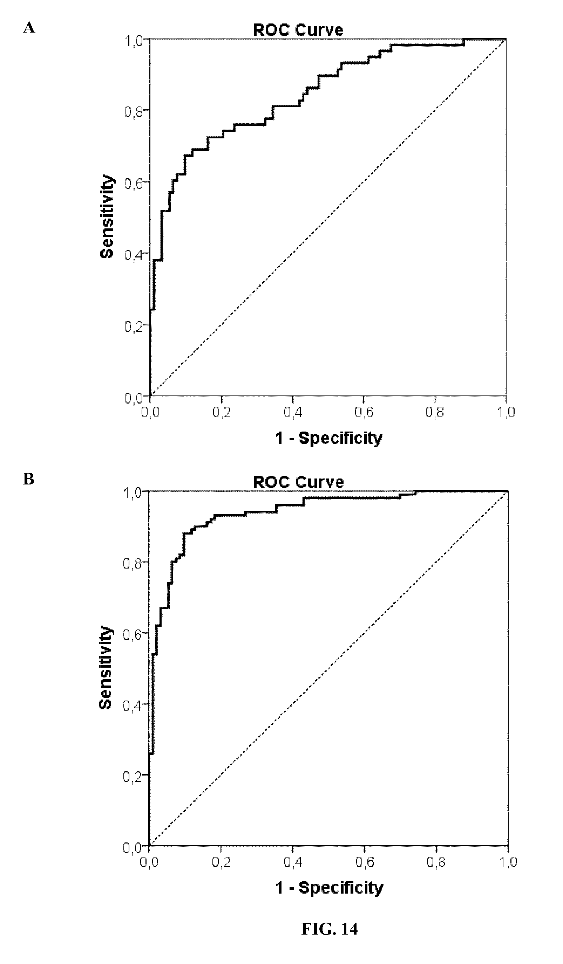

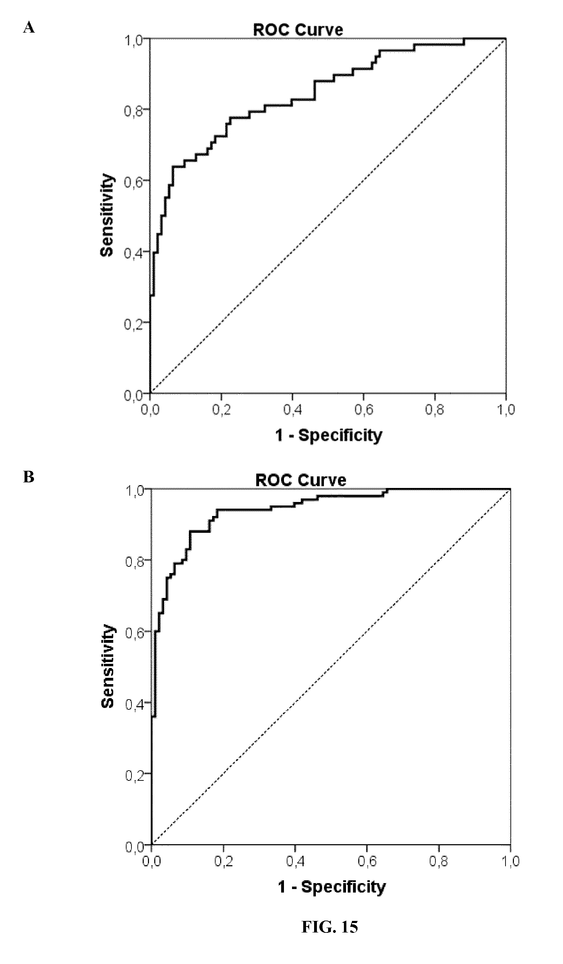

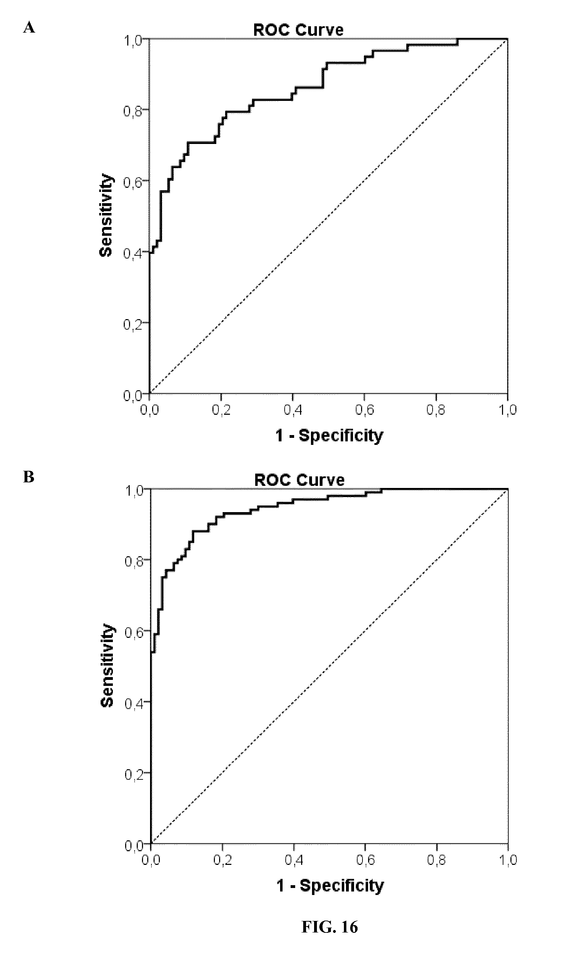

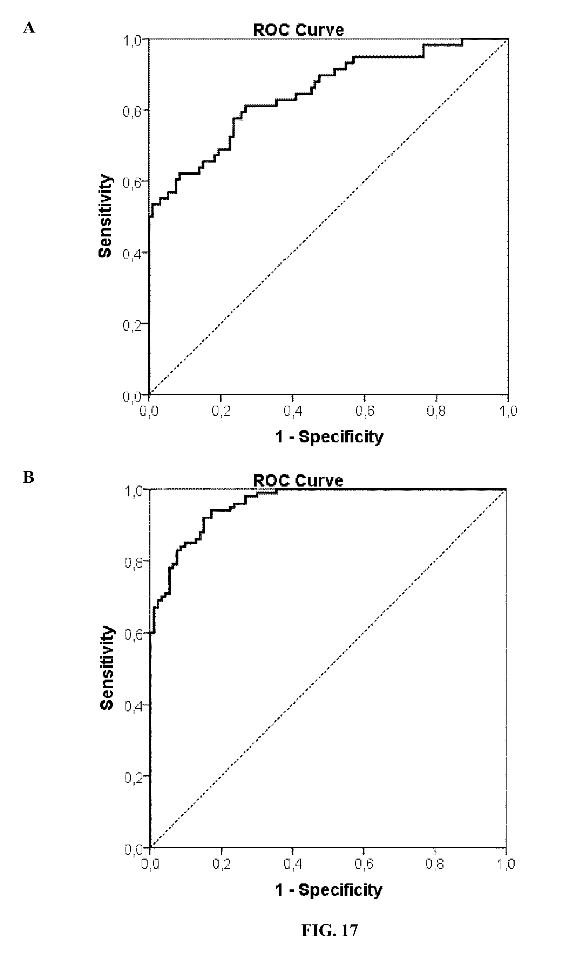

According to figures 1-17, it is evident that selected variables have a large impact on the ability to accurately classify individuals. Thus, "two-variable" model is able to discriminate aMCI and Alzheimer's disease from Healthy Control (AUC=0,724 and AUC=0,689, respectively) (table 9). As might be expected, using further number of variables (three-variable, figures 2-9; four variables, figures 10-12; five variables, figure 13; six variables, figure 14; and seven variables, figure 15) generates more robust analysis (higher AUC values) and, finally, "eight-variable" model and "nine-variable" model present top AUC values (figures 16 and 17) (table 9).

## Claims

1. An *in vitro* method for determining the likelihood that a patient with mild cognitive impairment develops Alzheimer's disease comprising
(a) determining in a sample from said patient the level of at least one biomarker selected from the biomarkers of Table 1, and
(b) comparing the level of said at least one biomarker with its reference value,
wherein decreased levels of at least one biomarker selected from the group consisting of 21:0 ceramide, apolipoprotein C-I, sarcosine, conjugated linoleic acid, docosahexaenoic acid, apolipoprotein C-II and glutamic acid compared to its reference value and/or increased levels of at least one biomarker selected from the group consisting of alanine and cortisol compared to its reference value are indicative of a high likelihood of the subject developing Alzheimer's disease.

2. The method according to claim 1, wherein the at least one biomarker is 21:0 ceramide.

3. The method according to claims 1 or 2 comprising determining at least two biomarkers selected from the biomarkers of Table 1.

4. The method according to claim 3 wherein the at least two biomarkers are 21:0 ceramide and apolipoprotein C-I.

5. The method according to claim 4 wherein the following combinations of markers are determined:
(i) 21:0 ceramide, apolipoprotein C-I and glutamic acid,
(ii) 21:0 ceramide, apolipoprotein C-I and alanine,
(iii) 21:0 ceramide, apolipoprotein C-I and cortisol,
(iv) 21:0 ceramide, apolipoprotein C-I and docosahexaenoic acid,
(v) 21:0 ceramide, apolipoprotein C-I and conjugated linoleic acid,
(vi) 21:0 ceramide, apolipoprotein C-I and sarcosine,
(vii) 21:0 ceramide, apolipoprotein C-I and apolipoprotein C-II,
(viii) 21:0 ceramide, apolipoprotein C-I, apolipoprotein C-II and cortisol,
(ix) 21:0 ceramide, apolipoprotein C-I, cortisol and glutamic acid,
(x) 21:0 ceramide, apolipoprotein C-I, cortisol and alanine,
(xi) 21:0 ceramide, apolipoprotein C-I, cortisol, glutamic acid and alanine,
(xii) 21:0 ceramide, apolipoprotein C-I, cortisol, glutamic acid, alanine and apolipoprotein C-II,
(xiii) 21:0 ceramide, apolipoprotein C-I, cortisol, glutamic acid, alanine, apolipoprotein C-II and conjugated linoleic acid,
(xiv) 21:0 ceramide, apolipoprotein C-I, cortisol, glutamic acid, alanine, apolipoprotein C-II, conjugated linoleic acid and docosahexaenoic acid.

6. The method according to any of claims 1 to 5 further comprising determining the presence or absence of apolipoprotein E type 4 isoform or a nucleic acid sequence encoding said apolipoprotein E type 4 isoform in a sample from said subject, wherein the presence of apolipoprotein E type 4 isoform or a nucleic acid sequence encoding said apolipoprotein E type 4 isoform in said sample is indicative of a high likelihood of the subject developing Alzheimer's disease.

7. An *in vitro* method for the diagnosis of Alzheimer's disease or mild cognitive impairment in a subject comprising
(a) determining in a sample from said subject the level of at least one biomarker selected from the biomarkers of Table 2, and
(b) comparing the level of said at least one biomarker with its reference value,
wherein decreased levels of at least one biomarker selected from the group consisting of 21:0 ceramide, apolipoprotein C-I, sarcosine, conjugated linoleic acid, docosahexaenoic acid and apolipoprotein C-II compared to its reference value and/or increased levels of the biomarker alanine compared to its reference value are indicative of the subject suffering from Alzheimer's disease or mild cognitive impairment.

8. The method according to claim 7 further comprising
(a) determining in a sample from said subject the levels of at least one additional biomarker selected from glutamic acid and cortisol and
(b) comparing the level of said additional biomarker with its reference value,
wherein decreased levels of glutamic acid compared to its reference value and/or increased levels of cortisol compared with its reference value are indicative of the subject suffering from Alzheimer's disease or mild cognitive impairment.

9. The method according to claims 7 or 8 wherein the at least one biomarker is 21:0 ceramide.

10. The method according to any of claims 7 to 9 comprising determining at least two biomarkers selected from the biomarkers of Table 2.

11. The method according to claim 10 wherein the at least two biomarkers are 21:0 ceramide and apolipoprotein C-I.

12. The method according to claim 11 wherein the following combinations of markers are determined:
(i) 21:0 ceramide, apolipoprotein C-I and glutamic acid,
(ii) 21:0 ceramide, apolipoprotein C-I and alanine,
(iii) 21:0 ceramide, apolipoprotein C-I and cortisol,
(iv) 21:0 ceramide, apolipoprotein C-I and docosahexaenoic acid,
(v) 21:0 ceramide, apolipoprotein C-I and conjugated linoleic acid,
(vi) 21:0 ceramide, apolipoprotein C-I and sarcosine,
(vii) 21:0 ceramide, apolipoprotein C-I and apolipoprotein C-II,
(viii) 21:0 ceramide, apolipoprotein C-I, apolipoprotein C-II and cortisol,
(ix) 21:0 ceramide, apolipoprotein C-I, cortisol and glutamic acid,
(x) 21:0 ceramide, apolipoprotein C-I, cortisol and alanine,
(xi) 21:0 ceramide, apolipoprotein C-I, cortisol, glutamic acid and alanine,
(xii) 21:0 ceramide, apolipoprotein C-I, cortisol, glutamic acid, alanine and apolipoprotein C-II,
(xiii) 21:0 ceramide, apolipoprotein C-I, cortisol, glutamic acid, alanine, apolipoprotein C-II and conjugated linoleic acid,
(xiv) 21:0 ceramide, apolipoprotein C-I, cortisol, glutamic acid, alanine, apolipoprotein C-II, conjugated linoleic acid and docosahexaenoic acid.

13. The method according to any of claims 7 to 12 further comprising determining the presence or absence of apolipoprotein E type 4 isoform or a nucleic acid sequence encoding said apolipoprotein E type 4 isoform in a sample from said subject, wherein the presence of apolipoprotein E type 4 isoform or a nucleic acid sequence encoding said apolipoprotein E type 4 isoform in said sample is indicative of the subject suffering from Alzheimer's disease or mild cognitive impairment.

14. The method according to any of claims 1 to 13 wherein the mild cognitive impairment is an amnestic mild cognitive impairment.

15. The method according to any of claims 1 to 14 wherein the Alzheimer's disease is prodromal Alzheimer disease.

16. The method according to any of claims 1 to 15 wherein the sample is blood, serum or plasma.

17. The method according to any of claims 1 to 16 implemented in a computer system or a computer system or computer program provided with means for implementing the methods according to any of claims 1 to 16, or a computer-readable data medium comprising said computer program.
